(19)

Europäisches Patentamt
European Patent Office
Office européen des brevets

(11) **EP 3 813 814 B1**

(12) **EUROPEAN PATENT SPECIFICATION**

(45) Date of publication and mention
of the grant of the patent:
**27.11.2024 Bulletin 2024/48**

(21) Application number: **19826435.0**

(22) Date of filing: **27.06.2019**

(51) International Patent Classification (IPC):
***C12Q 1/6883*** *(2018.01)* ***A61P 29/00*** *(2006.01)*

(52) Cooperative Patent Classification (CPC):
**G01N 33/6863; A61K 31/245; A61K 31/706;
A61K 31/7068; C12Q 1/6883;** C12Q 2600/154;
C12Q 2600/158; G01N 2800/52

(86) International application number:
**PCT/US2019/039411**

(87) International publication number:
**WO 2020/006175 (02.01.2020 Gazette 2020/01)**

(54) **METHODS AND COMPOSITIONS RELATING TO PERSONALIZED PAIN MANAGEMENT**

VERFAHREN UND ZUSAMMENSETZUNGEN IM ZUSAMMENHANG MIT PERSONALISIERTEM
SCHMERZMANAGEMENT

PROCÉDÉS ET COMPOSITIONS DE GESTION PERSONNALISÉE DE LA DOULEUR

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.06.2018 US 201862691019 P
14.09.2018 US 201862731548 P**

(43) Date of publication of application:
**05.05.2021 Bulletin 2021/18**

(73) Proprietor: **Children's Hospital Medical Center
Cincinnati, OH 45229-3039 (US)**

(72) Inventors:
• **CHIDAMBARAN, Vidya
Cincinnati, OH 45229 (US)**
• **JI, Hong
Cincinnati, OH 45229 (US)**

(74) Representative: **Forresters IP LLP
Skygarden
Erika-Mann-Straße 11
80636 München (DE)**

(56) References cited:
**US-A1- 2014 371 256**

• **VIDYA CHIDAMBARAN ET AL: "DNA methylation
at the mu-1 opioid receptor gene (<em>OPRM1</
em>) promoter predicts preoperative, acute, and
chronic postsurgical pain after spine fusion",
PHARMACOGENOMICS AND PERSONALIZED
MEDICINE, vol. Volume 10, 1 May 2017
(2017-05-01), pages 157 - 168, XP055739908, DOI:
10.2147/PGPM.S132691**
• **STEPHENS KIMBERLY E ET AL: "Associations
between genetic and epigenetic variations in
cytokine genes and mild persistent breast pain in
women following breast cancer surgery",
CYTOKINE, vol. 99, 29 July 2017 (2017-07-29),
pages 203 - 213, XP085271332, ISSN: 1043-4666,
DOI: 10.1016/J.CYTO.2017.07.006**
• **LIRK P ET AL: "Epigenetics in the perioperative
period", BRITISH JOURNAL OF
PHARMACOLOGY, WILEY-BLACKWELL, UK, vol.
172, no. 11, 27 April 2015 (2015-04-27), pages
2748 - 2755, XP071171503, ISSN: 0007-1188, DOI:
10.1111/BPH.12865**

**(Cont. next page)**

- MAUCK MATTHEW ET AL: "Epigenetics of chronic pain after thoracic surgery", CURRENT OPINION IN ANAESTHESIOLOGY., vol. 27, no. 1, 1 February 2014 (2014-02-01), US, pages 1 - 5, XP055891232, ISSN: 0952-7907, Retrieved from the Internet <URL:http://dx.doi.org/10.1097/ACO.0000000000000030> [retrieved on 20220215], DOI: 10.1097/ACO.0000000000000030
- YUAN SUN ET AL: "DNA Methylation Modulates Nociceptive Sensitization after Incision", PLOS ONE, vol. 10, no. 11, 4 November 2015 (2015-11-04), pages e0142046, XP055739889, DOI: 10.1371/journal.pone.0142046
- SI HAI-BO ET AL: "Correlations between inflammatory cytokines, muscle damage markers and acute postoperative pain following primary total knee arthroplasty", BMC MUSCULOSKELETAL DISORDERS, vol. 18, no. 1, 1 December 2017 (2017-12-01), XP055892470, Retrieved from the Internet <URL:https://www.ncbi.nlm.nih.gov/pmc/articles/PMC5473999/pdf/12891_2017_Article_1597.pdf> [retrieved on 20220215], DOI: 10.1186/s12891-017-1597-y
- ANONYMOUS: "GABA receptor - Wikipedia", 25 November 2021 (2021-11-25), XP055892601, Retrieved from the Internet <URL:https://en.wikipedia.org/wiki/GABA_receptor> [retrieved on 20220216]
- BUCHHEIT ET AL.: "Epigenetics and the Transition from Acute to Chronic Pain", PAIN MEDICINE, vol. 13, no. 11, 14 September 2012 (2012-09-14), pages 1474 - 1490, XP055514305
- SHRESTHA ET AL.: "Epigenetic Regulations of GABAergic Neurotransmission: Relevance for Neurological Disorders and Epigenetic Therapy", MED EPIGENET., vol. 4, no. 1, 10 February 2016 (2016-02-10), pages 1 - 19, XP055668147

**Description**

**TECHNICAL FIELD**

The present invention relates to the field of medicine, and more particularly to the field of pain and anxiety management, especially in the context of post-surgical pain.

**BACKGROUND**

Chronic postsurgical pain (CPSP) is often defined as pain that lasts beyond three months post- surgery, in the absence of other preexisting problems or postoperative complications (Macrae WA Brit. J. Anaesthesia 2008; 101(1):77-8). In children, the median prevalence of CPSP is 20% (Rabbitts JA et al., J. of Pain 2017; 18(6):605-614), however the incidence ranges from 11-54% after spine fusion (Chidambaran V et al., Eur. J. of Pain 2017; 21(7):1252-1265; Landman Z et al., Spine 2011; 36(10):825-829; Sieberg CB et al., J. of Pain 2013; 14(12):1694-1702) a painful surgery that adolescents undergo. CPSP is a classic example of gene-environment interaction, and involves multiple peripheral and central signaling and modulatory pathways regulated by genes (James SK Brit. J. of Pain 11(4):178-185). It has a heritable risk of 45%, (Young EE et al., J. Med. Genet. 2012; 49(1):1-9) and genetic factors explain some of the individual differences in pain perception (Angst MS et al., Pain 153(7):1397-1409; Norbury TA et al., Brain 2007; 130(11):3014-3049). However, a genetic basis for CPSP has been elusive (Katz J Pain 2012; 153(3):505-506) attributed partly to lack of replicability (Kim H et al., J. Pain 10(7):663-693) and inconsistent findings (Sadhasivam S et al., Pharmacogenomics 2012; 13(15):1719-1740) in genetic association studies (Branford R et al., Clin. Genet. 2012; 82(4):301-310; Walter C et al., Pain 2009; 146(3):270-275) and lack of consideration of gene-environmental interactions. Especially in children, caregiving environment and psychological factors like anxiety, prime children's pain responses, influence the 'epigenetic landscape' and influence his or her response to further surgical stress (Denk F et al., Neuron 2012; 73(3):435-444; Hettema JM et al., Arch. Gen. Psychiatry 2005; 62(2):182-189). Twin studies have shown that environmental factors are involved in the inter-personal differences in pain sensitivity (Angst MS et al., Pain 153(7):1397-1409). Elucidating gene-environmental influences through epigenetics is expected to explain critical gaps in predisposition and mechanisms involved in CPSP (Crow M et al., Genome Med. 2013; 5(2):12-21; Doerhing A et al., Eur. J. Pain 2011; 15(1):11-16).

DNA methylation via addition of a methyl group to the 5' position of a cytosine - guanine residue (CpG dinucleotide) is a common epigenetic mechanism associated with decreased transcriptional activity, and altered expression of nociceptive genes. It affects pain processing and the transition from acute to chronic pain (Bucheit T et al., Pain Medicine 2012; 13(11):1474-1490). We recently identified psychological, perioperative and $\mu$-opioid receptor gene (*OPRM1*) DNA methylation markers as predictors of acute and chronic postsurgical pain in adolescents undergoing spine fusion surgery. The *OPRM1* DNA methylation levels have been found to be elevated in opioid and heroin addicts (Chorbov VM et al., J. Opioid Manag. 2011; 7(4):258-264). Levenson et al., (USSN 12/631,622) developed a DNA methylation-based test for detecting and monitoring methylation states of biomarker genes which differ in the diseased compared to the non-diseased state (e.g., multiple sclerosis and breast cancer). However, effect sizes of single CpG sites are small, and sometimes identify associations that cannot be replicated. Hence, in this study, we use epigenome-wide association studies (EWAS) and a global bioinformatics-based approach to identify pathways, histone marks, and protein-DNA binding events enriched in DNA methylation differences associated with CPSP and anxiety. This approach integrates epigenetic-level data with biologic processes, pathways, and networks, and overcomes pitfalls of hypothesis-driven candidate marker association studies (Zorina-Lichenwalter K et al., Neuroscience 2016; 338:36-62). EWAS also allows novel candidate discovery, and have previously been used to study epigenetics of other conditions (e.g. panic disorder) (Shimada-Sugimoto M et al., Clinical Epigenetics 2017; 9(1):6-16) but not CPSP. We will test the hypothesis that shared biological processes enriched in DNA methylation will be associated with CPSP and anxiety, which will suggest new avenues for preventing and treating CPSP.

Chidmbaran, V. et al. Pharmacogenomics and Personalized Medicine, 2017; 10:157-168 teaches themethylation of the OPRMI gene as a predicator of acute and chronic postsurgical pain.

Therefore, there is a need to identify clinical markers for predicting a patient's susceptibility to CPSP in order to provide improved management of pain in the clinical setting.

**SUMMARY**

The invention is defined by the appendant claims.

The present invention is based, in part, on the discovery that methylation status in genes of certain molecular signaling pathways, especially those of the GABA receptor and Dopamine-DARPP32 Feedback in cAMP (hereinafter referred to simply as "dopamine-DARPP-32") signaling pathways, can be used as biomarkers for susceptibility to perioperative pain, and particularly CPSP. In addition, the disclosure provides certain cytokines whose expression in peripheral blood can also be used as a biomarker for susceptibility to pain, and for risk of developing CPSP. Cytokines whose protein expression is associated with CPSP include tumor necrosis factor alpha (TNFα) and interleukin-1RA (IL1RA) as well as fractalkine, epidermal growth factor (EGF), FMS-like tyrosine kinase 3 ligand (FLT-3L), macrophage derived chemokine (MDC), interleukin-13 (IL-13), interleukin-8 (IL-8), and interleukin-4 (IL-4). Accordingly, the disclosure provides but does not claim methods for pain management in the perioperative context, particularly through methods comprising assaying the DNA methylation status of certain genes and/or the expression of certain cytokines in peripheral blood, in order to identify a patient as susceptible to perioperative pain. The disclosure also provides but does not claim methods for treating a patient identified as susceptible to perioperative pain or one who is at risk of developing CPSP, for example by administering demethylating agent or an inhibitor of the repressor element-1 silencing transcription factor (REST), or by administering an anti-inflammatory agent where the patient presents with elevated levels of one or more of the cytokine biomarkers described herein.

In embodiments, the disclosure provides a method for the prophylaxis or treatment of perioperative pain in a patient in need thereof, the method comprising assaying, *in vitro,* a biological sample from the patient to determine the DNA methylation status of at least one CpG site in one or more of the human GABA-receptor and dopamine-DARPP-32 signaling pathway genes. In embodiments, the disclosure provides a method for identifying a patient who is susceptible to perioperative pain, the method comprising assaying, *in vitro,* a biological sample from the patient to determine the DNA methylation status of at least one CpG site in one or more of the human GABA-receptor and dopamine-DARPP-32 signaling pathway genes. In accordance with embodiments of the methods described here, the step of assaying a biological sample from the patient to determine the DNA methylation status of at least one CpG site in the GABA-receptor gene or in the dopamine-DARPP-32 signaling pathway includes detecting one or more 5-methylcytosine nucleotides in genomic DNA obtained from the sample. In embodiments, the step of assaying may further include one or more of isolating genomic DNA from the biological sample, treating the genomic DNA with bisulfite, and subjecting the genomic DNA to a polymerase chain reaction (DNA).

In embodiments, the perioperative pain is selected from preoperative pain, acute postoperative The invention concerns perioperative pain is chronic postoperative pain.

In embodiments, the at least one CpG site in the GABA-receptor gene or in the dopamine-DARPP-32 signaling pathway is identified in Tables 6A-6C.

In embodiments, the biological sample is a blood sample. For assaying DNA methylation, the blood sample is preferably a sample of whole blood, or one containing blood cells such as leukocytes and erythrocytes. In embodiments where the assay is for the expression of one or more cytokines in peripheral blood, the biological sample is preferably a serum sample.

In embodiments, a patient having a DNA methylation status of 'methylated' at the at least one CpG site is identified as a patient susceptible to perioperative pain or CPSP. In embodiments, a patient having a DNA methylation status of 'methylated' at the at least one CpG site is identified as a patient susceptible to CPSP or anxiety (as measured by the Childhood Anxiety Sensitivity Index (CASI). In embodiments, the patient identified as susceptible is administered a therapeutic agent selected from a demethylating agent and an inhibitor of the repressor element-1 silencing transcription factor (REST). In embodiments the agent is administered before or after a surgical procedure is performed on the patient. In embodiments, the demethylating agent is selected from procaine, zebularine and decitabine, or a combination of two or more of the foregoing. In embodiments, the demethylating agent is zebularine, decitabine, or a combination of two or more of the foregoing.

In embodiments, the biological sample is assayed by a method comprising isolation of genomic DNA from the biological sample, for example a sample of whole blood or serum. In embodiments, the biological sample is assayed by a method comprising, or further comprising, pyrosequencing. In embodiments, the pyrosequencing comprises two or more rounds of a polymerase chain reaction.

In embodiments, the patient is a female patient.

In embodiments, the patient is self-reported Caucasian or white.

In embodiments, the disclosure provides but does not claim a kit comprising a set of recombinant enzymes including one or more of DNA polymerase, ATP sulfurylase, luciferase, and apyrase, two substrates selected from one or both of adenosine 5' phosphosulfate (APS) and luciferin, at least one detectably labeled oligonucleotide primer designed to amplify in a polymerase chain reaction a DNA segment corresponding to at least one of the CpG sites defined in Table 6 and a methylated DNA polynucleotide of known sequence, as a positive control

**BRIEF DESCRIPTION OF THE DRAWINGS**

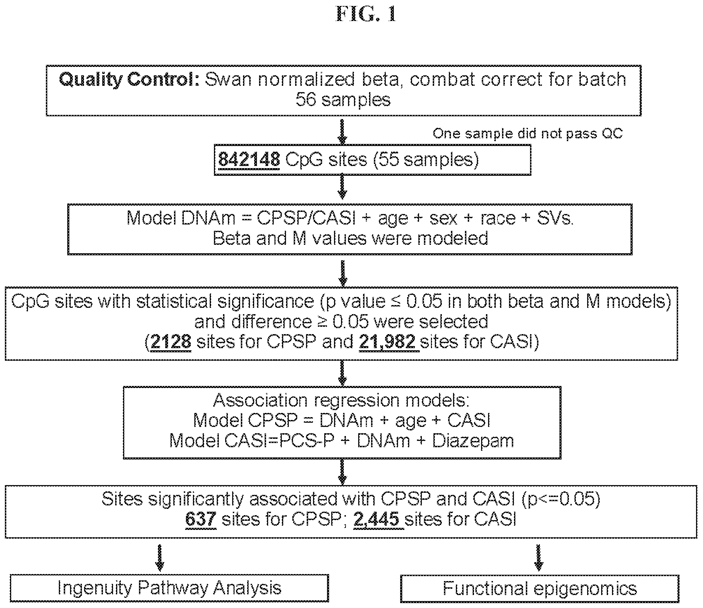

**Fig. 1**: Workflow for the statistical analysis of MethylationEPIC array data for chronic postsurgical pain (CPSP) and anxiety sensitivity (CASI) outcomes. DNA methylation beta and M values were modeled and CpG sites with methylation satisfying certain criteria were included in logistic (for CPSP) and linear (CASI) regression models adjusted for other covariates. Methylation sites significantly associated with outcomes were then included in the pathway and functional analyses.

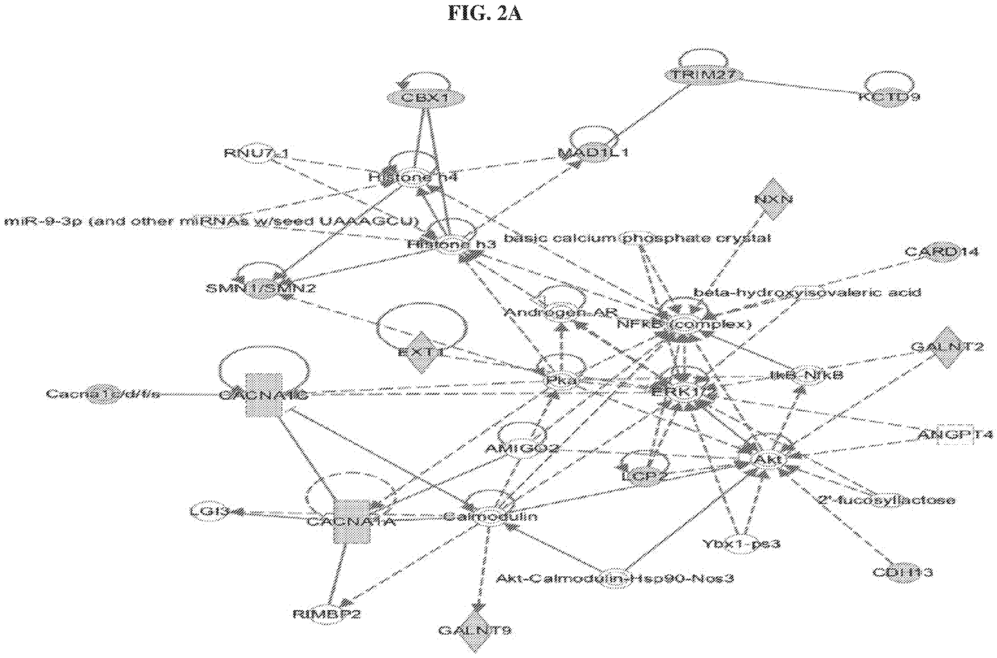

**Figs. 2A-2B**: Gene-gene interaction networks. Genes associated with the differentially methylated sites were uploaded to IPA. Based on P value cutoffs of $10^{-8}$, 3 networks were identified. Two of them were similar in function with several overlapping molecules. Hence, two of the different networks are presented here. The network in (**A**) is associated with cell signaling, molecular transport, and vitamin and mineral metabolism. It had a P-score of 33 and 14 focus molecules (including CACNA1A, CACNA1C, Calmodulin, ERK1/2, Histone h3, Histone h4, 1kB-NfkB, NfkB (complex), miR-9-3p). The network in (**B**) is associated with cell signaling, molecular transport, immune responses, metabolism and neurological diseases (p-value < 10-8), with a p-score of 12, and 6 focus molecules (including ESR1, KCNK6, PRIM2, and TNF).

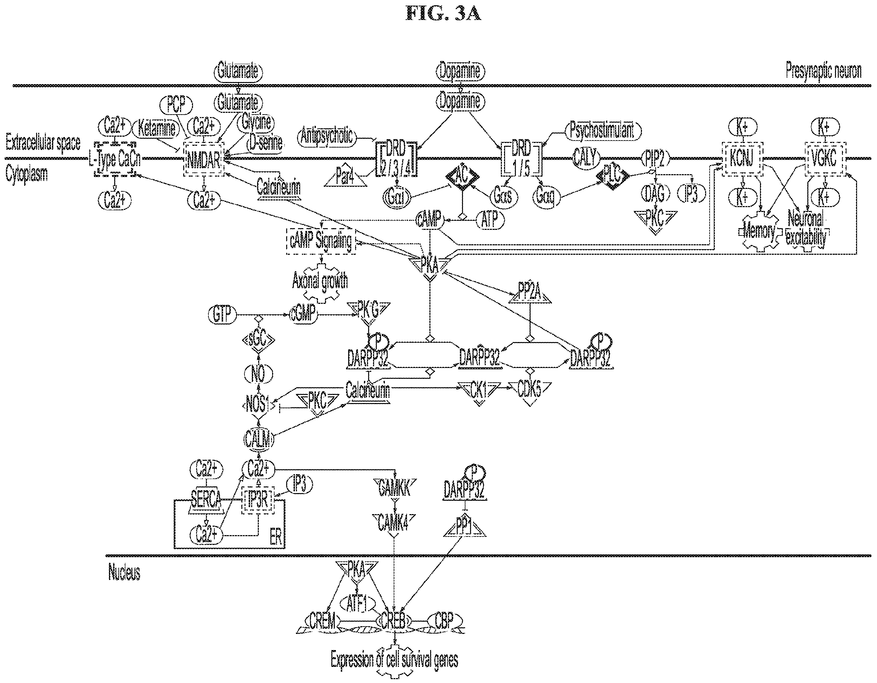

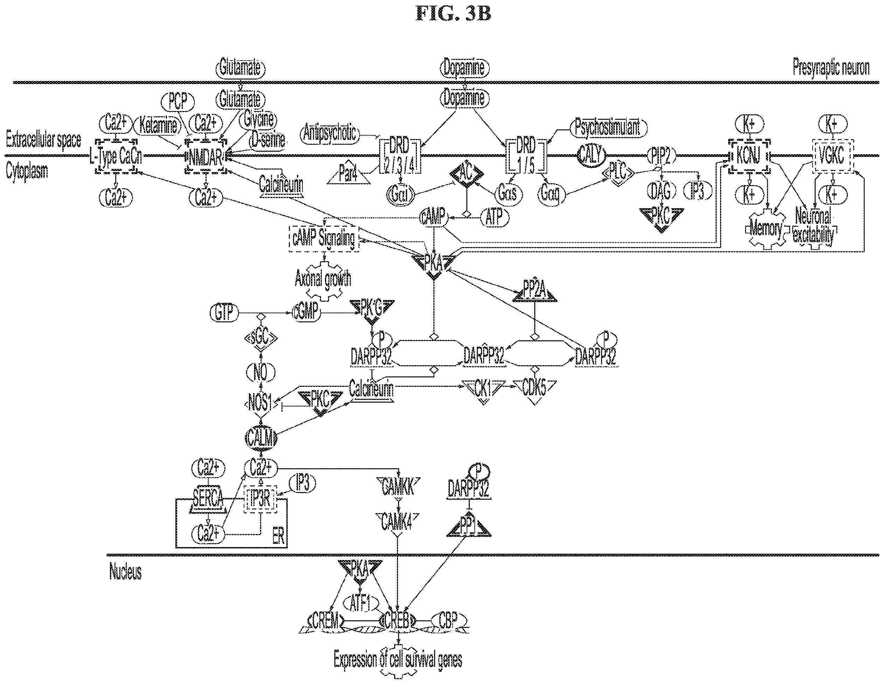

**Figs. 3A-3B:** DARPP32-Dopamine pathway analysis (generated from the Ingenuity Pathway Analysis software package (IPA) in the presynaptic neuron (one of the top canonical pathways shared by chronic postsurgical pain and anxiety outcomes). Ingenuity pathway illustration of the DARPP32-Dopamine pathway for CPSP (**A**) and Childhood Anxiety Sensitivity Index (CASI) (**B**). Some molecules/genes influenced by methylation changes in CPSP and CASI are common (calcium channel and adenyl cyclase) and some different (DARPP-32 protein, dopamine receptor, CAMKK in CPSP; and NMDA, K channel, PKA, CALM, and CREB for CASI). These molecules/genes have varied functions (channels, receptors, secondary messengers, etc.) and work together to regulate the pathway.

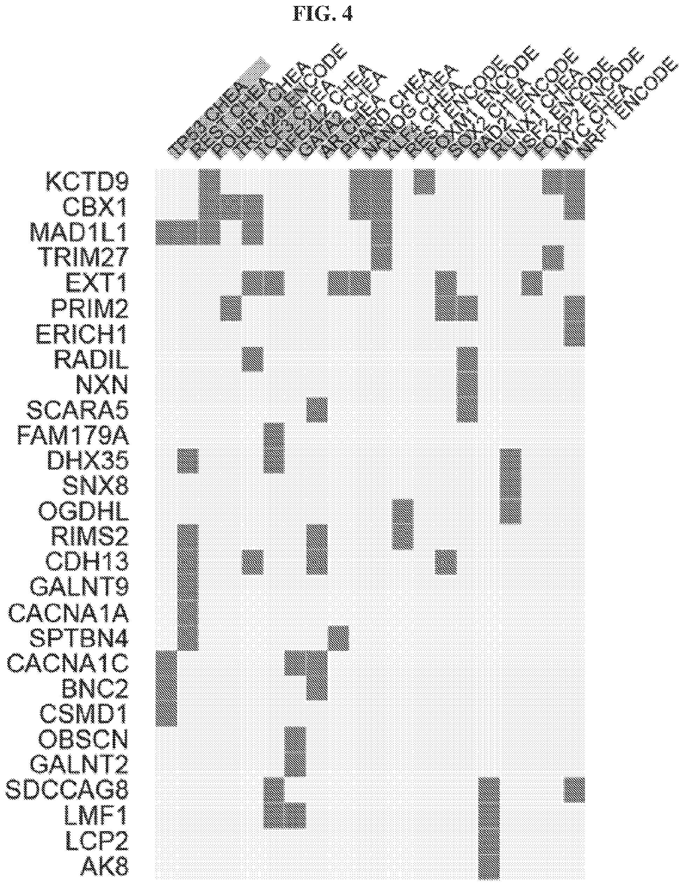

**Fig. 4:** Graphical depiction of the results of enrichment analysis using Enrichr. Transcription factors are listed across the top of the figure and target genes of the transcription factors are listed on the left side of the figure. Gray boxes in the figure indicate for each transcription factor where the target gene was also identified in our study. For example, for the REST transcription factor (here ENCODE mapped), target genes identified in our study include MAD1LI, DHX35, RIMS2, CDH13, GALNT9, CACNA1A and SPTBN4. Statistical significance is indicated by the gray vertical bars overlaid on the name of the transcription factor across the top of the figure (see also Table 7 *infra*). Transcription factors, from left to right, are: TP53, REST (CHEA), POU5F1, TRIM28, TCF3, NFE2L2, GATA2, AR, PPARD, NANOG, KLF4, REST(ENCODE), FOXM1, SOX2, RAD21, RUNX1, USF2, FOXP2, MYC, and NRF1.

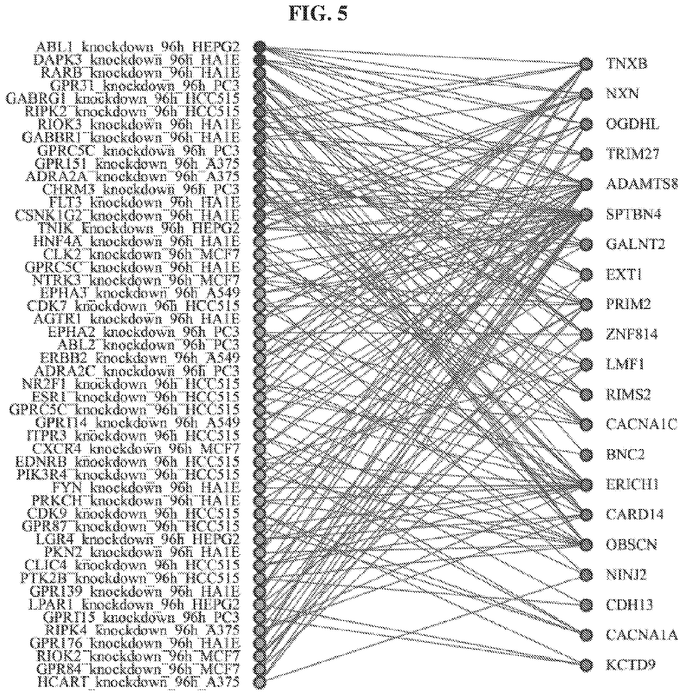

**Fig. 5:** Results of the LINCS library analyses are shown here. We use signatures of genetic knock-downs of gene-encoding protein kinases, which consist of genes whose mRNA expression is down-regulated in response to the loss of function for each kinase. Thus, genes identified in this study as undergoing epigenetic regulation (right side, gray circles) that are connected by edges to kinase knock-down signature nodes (left side, gray circles), where the shade of gray indicates the significance of the enrichment represent potential downstream targets of the respective kinase signaling cascade. Note that for each kinase, the corresponding cell line is also indicated, e.g., GABRG1 knock-down in HCC515 cells in the 5th row.

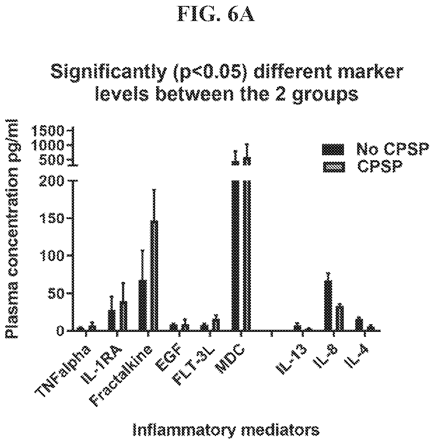

**Figs. 6A-C: A,** Average cytokine levels were measured in serum samples obtained at baseline and 4 time points within 2 hours of surgery (140 samples from 27 patients) using Human Cytokine/Chemokine Panel I (Milliplex® Immunology Panel). Bar graph shows average levels in patients who developed CPSP (N=14, right bar in each pair) and those who did not (No CPSP, left bar in each pair) are shown for cytokines having significantly different (p<0.05) levels between the two groups: TNFalpha, IL-1RA, Fractalkine, EGF, FLT-3L, MDC, IL-13, IL-8, and IL-4. **B-C,** DNA methylation (DNAm) of inflammatory genes IL-1B (**B**) and TNFalpha (**C**) at promoter CpG site correlates with decreased gene expression in blood. For **B**, $R^2$=0.87, p=0.021 and for **C**, $R^2$=0.80 and p=0.042. RT-PCR and pyrosequencing were used to examine DNAm and gene expression in blood for cytokine genes whose cytokines were elevated in CPSP.

## DETAILED DESCRIPTION

The present disclosure is based, in part, on associations between epigenetic modifications in the genomic DNA of certain genes and preoperative pain, acute postoperative pain, and chronic postoperative pain following surgery. These findings allow for the identification of patients who are likely to be particularly susceptible to perioperative pain, especially acute and chronic postoperative pain. The ability to identify such patients allows for the development of targeted prevention and treatment regimens for acute and chronic postoperative pain.

In the context of the present disclosure, the term "CpG site" refers to a site in genomic DNA where a cytosine nucleotide is followed by a guanine nucleotide when the linear sequence of bases is read in its 5 prime (5') to 3 prime (3') direction. The 'p' in "CpG" refers to a phosphate moiety and indicates that the cytosine and guanine are separated by only one phosphate group. A status of "methylated" in reference to a CpG site refers to methylation of the cytosine of the CpG dinucleotide to form a 5-methylcytosine.

In the context of the present disclosure, the terms "acute postoperative pain" and "chronic postoperative pain" are synonymous, respectively, with the terms "acute postsurgical pain" and "chronic postsurgical pain". The term "chronic postsurgical pain" may be abbreviated "CPSP". In this context, the term "chronic" refers to pain that persists for more than two or three months after surgery. Likewise, the term "acute" refers to pain occurring within the first two months after surgery.

In the context of the present disclosure, the term 'patient' refers to a human subject and a patient who is "susceptible" is one who is predisposed to suffering from perioperative pain, especially acute and chronic postsurgical pain. The identification of such patients according to the methods described herein is intended to provide for more effective personalized pain management and, in embodiments, for the targeted prevention and/or treatment of acute and/or chronic postsurgical pain. The patient identified as susceptible to perioperative pain or as susceptible to having an atypical perioperative anxiety response may be administered an agent to mitigate that susceptibility, such as a demethylating agent or an inhibitor of the repressor element-1 silencing transcription factor (REST). In embodiments, the demethylating agent may be selected from procaine, zebularine and decitabine, or a combination of two or more of the foregoing. In embodiments, the demethylating agent is zebularine, decitabine, or a combination of two or more of the foregoing.

In accordance with embodiments of the methods described here, the biological sample from the patient which is used to isolate genomic DNA and determine methylation status is a blood sample. In these embodiments, blood is used as a proxy for the target tissue, brain, because brain tissue is generally inaccessible in the clinical context in which the present methods are performed. The use of blood as a substitute for various target tissues has been validated for example, by ChIP assay findings showing similar transcription factors at the identified CpG sites across tissues and regulatory regions in brain tissue relevant to pain, which may be indicative of methylation at these sites having an effect on expression. Last but not least, methylation profiles derived from 12 tissues were compared in a previous study and found to be highly correlated between somatic tissues (Fan H et al., Zhonghua Yi Xue Yi Chuan Xue Za Zhi 2015; 32(5):641-645). Davies *et al.* also reported that inter-individual variation in DNA methylation was reflected across brain and blood, indicating that peripheral tissues may have utility in studies of complex neurobiological phenotypes (Davies MN et al., Genome Biol. 2012; 13(6):R43). For example, a comparison of methylation profiles of human chromosome 6 derived from different twelve tissues showed that CpG island methylation profiles were highly correlated (Fan S et al., Biochem. Biophys. Res. Comm. 2009; 383(4):421-425). More recently, some inter-individual variation in DNA methylation was found to be conserved across brain and blood, indicating that peripheral tissues such as blood can have utility in studies of complex neurobiological phenotypes (Davies MN et al., Genome Biol. 2012; 13(6):R43).

In accordance with embodiments of the methods described here, the methylation status at a genomic site, for example, at a CpG site as described herein, is binary, i.e., it is either methylated or unmethylated. In some embodiments where multiple CpG sites are assays, if at least one CpG site is methylated the region may be designated as methylated according to the claimed methods. This is because even if only one of several possible sites is methylated, if that site is a critical one for gene expression, its methylation may be sufficient. In other embodiments, where more than one of several possible CpG sites in a genomic region is methylated, the region may be designated as methylated or hypermethylated.

## Methods of Assaying DNA Methylation Status

Embodiments of the methods described here include assaying a patient's genomic DNA to determine the DNA methylation status at one or more CpG sites in a plurality of genes described *infra.*

As noted above, a status of "methylated" in reference to a CpG site refers to methylation of the cytosine of the CpG dinucleotide to form a 5-methylcytosine. Accordingly, methods of determining the DNA methylation status at one or more CpG sites in a genomic region of DNA generally involve detecting the presence of a 5-methylcytosine at the site, or multiple 5-methylcytosine in the region of interest. The determination of DNA methylation status can be performed by methods known to the skilled person. Typically such methods involve a determination of whether one or more particular sites are methylated or unmethylated, or a determination of whether a particular region of the genome is methylated, unmethylated, or hypermethylated, through direct or indirect detection of 5-methylcytosine at a particular CpG site, or in the genomic region of interest.

Whole-genome methylation can be detected by methods including whole-genome bisulfite sequencing (WGBS), high-performance liquid chromatography (HPLC) coupled with tandem mass spectrometry (LC-MS/MS), enzyme-linked immunosorbent assay (ELISA)-based methods, as well as amplification fragment length polymorphism (AFLP), restriction fragment length polymorphism (FRLP) and luminometric methylation assay (LUMA).

Generally, in the context of the methods described herein, the methylation status of one or more specific CpG sites is determined. Suitable methods may include bead array, DNA amplification utilizing a polymerase chain reaction (PCR) followed by sequencing, pyrosequencing, methylation-specific PCR, PCR with high resolution melting, cold-PCR for the detection of unmethylated islands, and digestion-based assays. Bisulfite conversion is typically an initial step in these methods. Accordingly, in embodiments, the method for assaying DNA methylation status in accordance with the present disclosure may include a step of bisulfite conversion, for example, a step of treating a sample of genomic DNA with bisulfite thereby converting cytosine nucleotides to uracil nucleotides except where the cytosine is methylated.

In embodiments, the step of assaying DNA methylation status comprises pyrosequencing. The analysis of DNA methylation by pyrosequencing is known in the art and can be performed in accordance with published protocols, such as described in Delaney *et al.,* (Delaney et al., In: Methods Mol. Biol. Ed. Albert C. Shaw, 2015; vol. 1343 (Chapter 19): pp. 249-264; Springer Humana Press, NY). This technique detects single-nucleotide polymorphisms which are artificially created at CpG sites through bisulfite modification of genomic DNA, which selectively converts cytosine to uracil except where the cytosine is methylated, in which case the 5-methylcytosine is protected from deamination and the CG sequence is preserved in downstream reactions. Generally, the method comprises treating extracted genomic DNA with bisulfite and amplifying the DNA segment of interest with suitable primers, *i.e.*, using a PCR-based amplification.

**Demethylating Agents**

DNA demethylating agents inhibit DNA methyltransferases (DNMTs) such as DNMT1, which is responsible for the maintenance of methylation patterns after DNA replication, DNMT3A, and DNMT3B, each of which carries out *de novo* methylation.

In accordance with certain embodiments of the methods described here, a patient identified as susceptible to perioperative pain and anxiety based on the patient's methylation status as described herein may be administered one or a combination of two or more demethylating agents, for example, as part of a personalized pain management regimen.

In embodiments, a demethylating agent administered in accordance with embodiments of the methods described here may be a nucleoside-like DNMT inhibitor or a non-nucleoside DNMT inhibitor.

In an embodiment, the agent is a nucleoside-like DNMT inhibitor. In embodiments, the nucleoside-like DNMT inhibitor is selected from azacytidine (VIDAZA™), and analogs thereof, including 5-aza-2'-deoxycytidine (decitabine, 5-AZA-CdR), 5-fluoro-2'-deoxycytidine, and 5,6-dihydro-5-azacytidine. In embodiments, the nucleoside-like DNMT inhibitor is selected from pyrimidine-2-one ribonucleoside (zebularine).

In an embodiment, the agent is a non-nucleoside-like DNMT inhibitor. In embodiments, the agent is an antisense oligonucleotide. In embodiments, the antisense oligonucleotide is MG98, a 20-base pair antisense oligonucleotide that binds to the 3' untranslated region of DMNT1, preventing transcription of the DNMT1 gene. In embodiments, the non-nucleoside-like DNMT inhibitor is RG108, a small molecule DNA methylation inhibitor (Graca I et al., Curr. Pharmacol. Design. 2014 20:1803-11).

**REST Inhibitors**

In accordance with embodiments of the methods described here, a patient identified as susceptible to perioperative pain based on the patient's methylation status as described herein, including a patient identified as susceptible to perioperative pain or hyperalgesia, may be administered an inhibitor of the repressor elements-1 silencing transcription factor (REST). In embodiments, the REST inhibitor is denzoimidazole-5-carboxamide derivative (X5050) (Charbord J et al., Stem Cells 2013; 20:1803-1811).

**Target Population**

In embodiments of the methods described here, the methods are directed to a target population of patients in need of prophylaxis or treatment of perioperative pain. In embodiments, the target patient population may be further defined as discussed below. In the context of the methods described here, the term "patient" refers to a human subject. In embodiments, the term may more particularly refer to a human subject under the care of a medical professional.

In embodiments, the target patient population may be further defined by sex, age, or self-reported human population or ethnic group. For example, in embodiments the patient is a female. In embodiments, the patient is an adolescent, as that term is understood by the skilled medical practitioner. In embodiments, the patient's race or ethnicity is self-reported as white or Caucasian.

Kits

Kits useful in the methods disclosed here comprise components such as primers for nucleic acid amplification, hybridization probes, means for analyzing the methylation state of a deoxyribonucleic acid sequence, and the like. The kits can, for example, include necessary buffers, nucleic acid primers, and reagents for detection of methylation, as well as suitable controls, including for example bisulfite conversion controls, such as a bisulfite-treated DNA oligonucleotide of known sequence, and template free negative controls for pyrosequencing, as well as necessary enzymes (*e.g.* DNA polymerase), and suitable buffers.

In some embodiments, the kit comprises one or more nucleic acids, including for example PCR primers and bisulfite-treated DNA for use as a control, for use in the detection of the methylation status of one or more of the specific CpG sites identified herein, as well as suitable reagents, *e.g.*, for bisulfite conversion, for amplification by PCR and/or for detection and/or sequencing of the amplified products.

In embodiments, the kit comprises a set of PCR primers for detecting the methylation status of one or more of the CpG sites identified herein. In embodiments, the kit comprises at least two sets of primers, long and nested.

In certain embodiments, the kit further comprises a set of instructions for using the reagents comprising the kit.

Unless defined otherwise, technical and scientific terms used herein have the same meaning as commonly understood by one of ordinary skill in the art to which this invention belongs. Singleton et al., Dictionary of Microbiology and Molecular Biology 3rd ed., J. Wiley & Sons (New York, NY 2001); March, Advanced Organic Chemistry Reactions, Mechanisms and Structure 5th ed., J. Wiley & Sons (New York, NY 2001); and Sambrook and Russel, Molecular Cloning: A Laboratory Manual 3rd ed., Cold Spring Harbor Laboratory Press (Cold Spring Harbor, NY 2001) provide one skilled in the art with a general guide to many of the terms used in the present application.

## EXAMPLES

### Methods

A prospective observational cohort study was conducted in 73 adolescents with idiopathic scoliosis undergoing posterior spine fusion under standard anesthesia (Propofol-remifentanil total intravenous anesthesia) and postoperative analgesia (Patient Controlled Analgesia (PCA) plus scheduled intravenous acetaminophen, ketorolac, diazepam as needed and methocarbamol) protocols. The study was approved by the Cincinnati's Children's Hospital Medical Center (CCHMC) institutional review board. This study was registered with Clinicaltrials.gov identifiers NCT01839461 and NCT01731873. Written informed consent was obtained from parents, and assent was obtained from children before enrollment.

### Participants

Healthy non-obese subjects with an American Society of Anesthesiologists (ASA) physical status less than or equal to two (mild systemic disease), aged ten to 18 years, with a diagnosis of idiopathic scoliosis and/or kyphosis, undergoing elective spinal fusion were recruited. Exclusion criteria included pregnant or breastfeeding females, presence of chronic pain defined as use of opioids in the past six months, liver or renal diseases and developmental delays.

### Data Collection

Preoperatively, data regarding demographics (sex, age, race), weight, pain scores (numerical rating scale/0-10 NRS) and home medication use were obtained. Anxiety scores for both child and a parent were assessed using the 0-10 visual analog scale (VAS), a simple validated scale which has been used previously in children. Questionnaires were administered as described in Table 1. Intraoperative data collected included propofol and remifentanil doses, duration of surgery, and number of vertebral levels fused. In the immediate postoperative period (postoperative days (POD) one and two), pain scores (every four hours), morphine and diazepam doses administered were noted. After hospital discharge, questionnaires were administered over phone/email in a standard fashion, per schedule presented in Table 1 to obtain psychosocial and pain measures.

Table 1. Data collection scheme

| Data variants | Preoperative | Intraoperative | >48 hours after surgery | 10-12 months after surgery |
|---|---|---|---|---|
| Demographics VAS Anxiety scores (parent and child) Pain score (child) | X | | | |
| Surgical duration Vertebral levels fused Propofol dose Remifentanil dose | | X | | |
| Pain scores Opioid consumption Diazepam use Analgesic adjuncts | X | | X | X |
| Child Questionnaires CASI Pain assessment | X | | | |

(continued)

| Data variants | Preoperative | Intraoperative | >48 hours after surgery | 10-12 months after surgery |
|---|---|---|---|---|
| Parent Questionnaires PCS-P | X | | | |

**Notes**: Time is calculated from end of surgery. X indicates the phase in which the data is collected. **Abbreviations**: VAS: Visual Analog Scale; CASI: Childhood Anxiety Sensitivity Index; PCS-P, Pain Catastrophizing Scale (parent version); PCS-P, pain catastrophizing scale (parent version).

## Outcomes

Outcomes evaluated were: a) CPSP, defined as NRS>3/10 at 10-12 months post-surgery (Macrae and Davies, Seattle: IASP Press; 1999). These cut-offs were used because NRS pain scores >3 (moderate/severe pain) have been described as a predictor for persistence of pain and associated with functional disability (Gerbershagen et al., Brit. J. Anaesth. 2011, 107(4):619-629); and b) Child Anxiety Sensitivity Index (CASI), an 18-item self-report tool designed to measure symptoms of anxiety in children and adolescents, with total scores ranging from 18-54, was chosen as the anxiety measure, because CASI is strongly correlated with state and trait anxiety (Rabian et al., J. Clin. Child Psychol. 1999; 28(1):105-112). It is a measure of the degree to which one interprets anxiety-related symptoms as being associated with potentially harmful somatic, psychological, or social consequences (Silverman et al., J. Abnorm. Psychol. 2003; 112(3): 364-374). The CASI has demonstrated high internal consistency in both clinical and nonclinical samples (aged 8-15.8 years), good test-retest reliability and good construct validity (Silverman et al., J. Abnorm. Psychol. 2003; 112(3): 364-374). Our own studies have shown that the odds of pain persistence at 1 year after spine surgery was 1.24 higher for every unit increase in CASI score (95% CI 1.09-1.42, p=0.002) (Chidambaran et al., 2017; Pharmgenomics Pers Med. 10: 157-168) and is supported by studies in other pediatric cohorts (Page et al., 2013; J. Pain Res 6:167-180).

## Measurement of DNA methylation

Blood was drawn upon intravenous line placement before surgery, from which DNA was isolated on the same day and frozen at -20°C. To study DNA methylation, 500 ng of genomic DNA of acceptable quality (measured by Thermo Scientific NanoDrop spectrophotometer and with a 260/280 ratio ranging from 1.6 to 2.0) was extracted, treated with bisulfite using Zymo EZ DNA Methylation Gold kit (Zymo Research, Orange, CA, USA), according to the manufacturer's instructions. DNA methylation was analyzed using the Infinium MethylationEPIC kit (©Illumina Inc., San Diego, CA) which provides unparalleled coverage of CpG islands, genes, and enhancers.

## Data analysis

Demographics and patients' clinical characteristics were summarized as mean (with standard deviation), median (IQR) and frequency (percentage) according to the distribution of the data. Prior to the DNA methylation analysis, the quality of the methylation arrays was assessed using sample-independent and -dependent internal control probes included on the array for staining, extension, hybridization, specificity and bisulfite conversion. The number of probes with detection P value $\leq 0.05$ was examined for each sample. Only samples that passed the quality control with >95% probes detected were included in the analysis. CpG sites that were not detected in all samples at p=0.01 level or located on the X and Y chromosomes were excluded. The signal intensities were background-adjusted using out-of-band probes (noob), and normalized using subset-quantile within array normalization (swan in R package 'minfi'). Beta values, calculated as

$$beta = \frac{signal_{methylation}}{signal_{methylation} + signal_{unmethylation}}$$

, and M values , the logit transformation of the beta values (Du P et al., BMC Bioinformatics 2010; 11:587) were used. Surrogate variable analysis (SVA) was used to control batch effect and unknown confounders such as cell composition. For each of the CpG sites, the association of DNAm with CPSP and CASI was tested with linear regression. Age, sex, race and significant surrogate variables were adjusted. CpG sites whose DNAm (both beta and M values) were associated with CPSP or CASI at p$\leq$0.05 level were selected for further evaluation. The selected sites should also have differences $\geq 0.05$ in beta between CPSP yes and no groups. As impact of non-genetic covariates were previously found on CPSP and CASI (Chidambaran V et al., Eur. J. Pain 2017; 21(7):1252-1265) to ensure the robustness of the association identified from the above analyses in which the DNAm was used as the dependent variable, we conducted logistic and linear regression for CPSP and CASI, respectively, in which CPSP and CASI were used as dependent variables, beta value as primary independent variable, and significant non-genetic co-variables were adjusted. Significant non-genetic co-variables were identified by univariate analysis for CPSP (factors tested: age, sex, race, morphine dose in mg/kg POD1 and POD2, preoperative anxiety score (VAS) for child and parent, duration of surgery, vertebral levels fused, PCS-P and CASI) and CASI (factors tested: age, sex, race, PCS-P, diazepam doses and parent anxiety score), and selection of co-variables associated at p < 0.10. Analyses were performed using Statistical Analysis System (SAS), version 9.4 (SAS Institute Inc., Cary, NC) and R. Only CpG sites showing significance association with beta values in these models were extracted from MethylationEPIC array annotation files and imported into Ingenuity Pathway Analysis software (IPA, Ingenuity Systems, Redwood City, CA) for pathway mapping, gene network detection, and upstream regulator identification.

To identify potential regulatory mechanisms altered by CpG methylation differences, we evaluated CpG sites that were significant in the previous step (p<0.05) against a control set of CpG sites (p>0.4) using a compiled large collection of functional genomics datasets from various sources, including ENCODE (Consortium EP et al., Nature 2012; 489(7414):57-74), Roadmap Epigenomics (Bernstein BE et al., Nat. Biotech. 2010; 28(10):1045-1048), Cistrome (Liu T et al., Genome Biol. 2011;12(8):R83), and ReMap-ChIP (Griffon A et al., Nucl. Acids Res. 2015; 43(4):e27). In total, this database contains 4,045 datasets performed in 1,069 different cell types and conditions. Monitoring of protein binding interactions of 1,544 data sets including transcription factors with the human genome was performed using ChIP-seq. A particular histone mark was measured in 1,213 data sets using ChIP-seq.; 277 data sets measured open chromatin through DNase-seq; 55 data sets measured expression-quantitative loci (eQTLs); and 558 data sets predicted "Active-Chromatin" states using combinations of histone marks (Ernst J et al., Nature 2011; 473(7345)43-49). Collectively, 240 of these experiments were performed in brain-related cell lines and cell types.

We next used the RELI algorithm to estimate the statistical enrichment of histone marks and protein-binding events at the genomic loci displaying altered DNA methylation (Harley JB et al., Nature Neurosci. 2018; 17(2):192-200). As input, the method took a set of genomic loci (in this case, regions with differential methylation marks). The coordinates of each locus were padded by 100 bases in either direction to account for experimental resolution. The resulting loci were then systematically intersected with the ChIP-seq and the epigenetic data set libraries described above, and the number of input regions overlapping each dataset by at least one base was counted. Next, a P-value describing the significance of this overlap was estimated using a simulation-based procedure. To this end, the control set of CpG sites that do not change (p>0.4) was used as a negative, background set. A distribution of expected overlap values was then created from 2,000 iterations of randomly sampling from the negative set, each epic choosing a set of negative examples that match the input set in terms of the total number of genomic loci and the length of each locus. The distribution of the expected overlap values from the randomized data resembles a normal distribution, and can thus be used to generate a Z-score and corresponding P-value estimating the significance of the observed number of input regions that overlap each data set. Collectively, this procedure controlled for the count and sizes of the input loci, and the count and sizes of each individual dataset in the library. The final output of the method is a p-value based ranking of all of the functional genomics datasets, in terms of their overlap with the input set.

With the goal of further elucidating pathways and potential regulatory mechanisms underlying the observed epigenetic changes, we performed enrichment analysis using a comprehensive, curated library of transcription factor targets that combines results from ENCODE and literature based CHEA ChIP-seq experiments, available through Enrichr (http://amp.pharm.mssm.edu/Enrichr/). Next, we used the Library of Integrated Network-based Cellular Signatures (LINCS) of genetic perturbations (gene knockdowns of the 39 genes common to both outcomes with DNA methylation changes) and connectivity analysis, with the focus on kinase signaling pathways, available through Pinet (http://pinet-server.org) and Enrichr (Koleti A et al., Nucl. Acids Res. 2018; 46(D1):D558-D566). One of the goals of LINCS library is to enable analysis of connectivity between genetic (Keenan AB et al., Cell System. 2018; 6(1):13-24) and chemical perturbations by measuring correlations between their transcriptional echo (correlation between landmark gene expression vectors). Here, we use signatures of genetic knock-downs of gene encoding protein kinases, which consist of genes whose mRNA expression is downregulated in response to the loss of function for each kinase.

## Results

The mean age of participants was 14.4 years (SD 1.6); they were mostly white (82%) and female (84%). Demographics and description of variables evaluated, are presented in Table 2. Median preoperative pain score was 0.0 (IQR 0.0-1.0) and mean (SD) for AUC on POD1 and POD2 was 202.6 (84.3). As expected, there was a significant difference in NRS pain scores at 6-12 months between the non-CPSP (0.0 (0.0-1.0)) and CPSP (5.0 (4.0-6.0)) groups (p<0.001). Of 73 subjects recruited, follow-up for CPSP outcomes was successful for 56 subjects. Incidence of CPSP in this cohort was 15/56 (29%).

At a significance threshold of p<0.1, univariate analyses identified age and CASI were significant determinants of CPSP (p= 0.070 and 0.090 respectively) (Table 2); and PCS-P (p=0.015) and diazepam dose (p=0.090) for CASI. Preoperative pain, AUC and pain at 6-12 months were all significantly higher in the CPSP group compared to the non-CPSP group (p=0.006, 0.002 and <0.001 respectively). Since preoperative pain, AUC and CPSP are correlated pain outcomes, with possible overlap of DNA methylation associations, we did not include them as co-variables in the multivariate model for CPSP.

Table 2. Demographics and other variables

| | All | Chronic Post-surgical pain (CPSP)* | | | CASI (N=56) | |
|---|---|---|---|---|---|---|
| | N=73 | No (N=40) | Yes (N=15) | p value | [d]Correlation coefficient | p value |
| [a]Age (years) | 14.4 ± 1.6 | 14.3 ± 1.8 | 15.2 ± 1.3 | 0.07 | 0.01 | 0.96 |
| [b]Sex (Male) | 9 (16%) | 8 (20%) | 1 (7%) | 0.42 | 0.06 | 0.68 |
| [b]Race (White) | 45 (82%) | 33 (83%) | 12 (80%) | 1.00 | 0.04 | 0.76 |
| [c]Weight (Kg) | 53.7 (50.4-58.0) | 53.5 (50.4-59.8) | 53.9 (51.0-58.0) | 0.84 | -0.19 | 0.25 |
| [c]VAS Anxiety (Child) | 4.4 (3.0-6.9) | 4.4 (3.0-6.8) | 3.3 (3.0-8.5) | 0.75 | 0.26 | 0.14 |
| [c]VAS Anxiety (Parent) | 6.7 (4.7-8.1) | 5.4 (4.4-8.0) | 7.6 (5.0-8.8) | 0.37 | 0.25 | 0.15 |
| [c]Number of vertebral levels fused | 12.0 (10.0-13.0) | 12.0 (11.0-13.0) | 12.0 (10.0-12.0) | 0.40 | -0.01 | 0.95 |
| [a]Surgical duration (hours) | 4.2 ± 1.1 | 4.3 ± 1.0 | 3.9 ± 1.4 | 0.38 | -0.12 | 0.47 |
| [c]Morphine dose POD1&2 mg/kg | 1.2 (0.9-1.7) | 1.2 (1.0-1.8) | 1.7 (0.9-2.9) | 0.13 | 0.15 | 0.27 |
| [c]CASI | 28.4 (24.0-32.5) | 27.0 (24.0-31.0) | 29.8 (26.0-34.3) | 0.09 | - | - |
| [c]pain scores at 6-12 months | 1.0 (0.0-4.0) | 0.0 (0.0-1.0) | 5.0 (4.0-6.0) | <0.0 01 | 0.15 | 0.38 |
| [a]PCS-P | 21.7 ± 11.6 | 20.4 ± 12.5 | 24.1 ± 9.7 | 0.48 | 0.40 | 0.015 |
| [c]Diazepam use mg/kg | 0.1 (0.1-0.2) | 0.1 (0.1-0.2) | 0.2 (0.1-0.2) | 0.12 | 0.26 | 0.09 |

Note: a: data exhibited normal distribution; shown as mean ± SD and compared using t tests for PP.
b: shown as frequency (proportion) and compared using Fisher's exact tests for PP.
c: data did not exhibit a normal distribution; shown as median (IQR) and compared using Wilcoxon rank sum tests for PP.
d: Spearman correlation coefficient.
*Data from 55 subjects who had CPSP outcomes and evaluable MethylationEPIC array data are presented here
Abbreviations: VAS: Visual Analog Scale; POD: Postoperative Day; CASI: Childhood anxiety sensitivity index;
PCS-P: Pain catastrophizing scale -parents

**DNA methylation and CPSP/CASI association analyses**

Of the 73 samples, one was excluded from analysis due to bad array quality. The remaining samples all had more than 99% of the probes detected. For CPSP, a final set of 637 CpG sites were selected; for CASI, 2,445 CpG sites were selected for IPA analyses (Fig. 1). The distribution of differentially methylated regions in association with CPSP and the CASI (*P*< .05 and delta-beta > 5) with regard to genomic location, are presented in Table 3.

Table 3. Distribution of differentially methylated regions associated with CPSP and anxiety sensitivity (CASI) based on genomic location.

| | CPSP Differentially Methylated Sites | | CASI Differentially Methylated Sites | |
|---|---|---|---|---|
| Genomic Location | No. | % | No. | % |
| TSS1500 | 211 | 11.01 | 103 | 11.77 |
| TSS200 | 93 | 4.85 | 35 | 4.00 |

(continued)

|  | CPSP Differentially Methylated Sites | | CASI Differentially Methylated Sites | |
|---|---|---|---|---|
| Genomic Location | No. | % | No. | % |
| 5'UTR | 40 | 2.09 | 64 | 7.31 |
| First exon | 17 | .89 | 2 | .23 |
| Gene body | 729 | 38.03 | 318 | 36.24 |
| 3'UTR | 3 | .16 | 22 | 2.51 |
| N_Shelf | 31 | 1.62 | 14 | 1.60 |
| N_Shore | 57 | 2.97 | 18 | 2.06 |
| S_Shelf | 24 | 1.25 | 12 | 1.37 |
| S_Shore | 42 | 2.19 | 19 | 2.17 |
| CpG island | 117 | 6.10 | 29 | 3.31 |
| Open sea | 553 | 28.85 | 239 | 27.31 |

**Pathway enrichment analyses**

To assess the possible overall influence of the significant differences in DNA methylation enrichment for CPSP and CASI, two pathway analyses were performed. Annotation information on the significantly associated CpG sites was used for the analysis. In total, 310 genes (CPSP) and 1,526 genes (CASI) were annotated to the CpG sites. The top canonical pathways mapped to significantly methylated CpG sites for CPSP and CASI and overlap with gene sets defining the pathways at a p-value<0.05 are shown in Table 4.

Table 4. Top canonical pathways for CPSP and CASI

| Ingenuity Canonical Pathways for Chronic postsurgical pain | -log(p-value)* |
|---|---|
| GABA Receptor Signaling | 3.78 |
| PKCθ Signaling in T Lymphocytes | 3.11 |
| Dopamine-DARPP32 Feedback in cAMP Signaling | 2.39 |
| Cellular Effects of Sildenafil (Viagra) | 2.26 |
| GPCR-Mediated Nutrient Sensing in Enteroendocrine Cells | 1.92 |
| Calcium Signaling | 1.87 |
| nNOS Signaling in Skeletal Muscle Cells | 1.87 |
| Dopamine Receptor Signaling | 1.83 |
| FcγRIIB Signaling in B Lymphocytes | 1.80 |
| cAMP-mediated signaling | 1.66 |
| Corticotropin Releasing Hormone Signaling | 1.55 |
| Fatty Acid α-oxidation | 1.53 |
| Tryptophan Degradation X (Mammalian via Tryptamine) | 1.43 |
| **Top 20 Ingenuity Canonical Pathways for Childhood anxiety sensitivity** | **-log(p-value)*** |
| Cardiac β-adrenergic Signaling | 8.61 |
| cAMP-mediated signaling | 5.67 |
| CDK5 Signaling | 5.54 |
| Protein Kinase A Signaling | 5.22 |
| G-Protein Coupled Receptor Signaling | 4.58 |
| Dopamine-DARPP32 Feedback in cAMP Signaling | 4.47 |

(continued)

| Top 20 Ingenuity Canonical Pathways for Childhood anxiety sensitivity | -log(p-value)* |
|---|---|
| Axonal Guidance Signaling | 4.13 |
| Dopamine Receptor Signaling | 4.12 |
| GNRH Signaling | 3.98 |
| Androgen Signaling | 3.89 |
| Cellular Effects of Sildenafil (Viagra) | 3.71 |
| Nitric Oxide Signaling in the Cardiovascular System | 3.63 |
| G Beta Gamma Signaling | 3.58 |
| mTOR Signaling | 3.43 |
| Melanocyte Development and Pigmentation Signaling | 3.41 |
| PTEN Signaling | 3.35 |
| AMPK Signaling | 3.30 |
| GPCR-Mediated Integration of Enteroendocrine Signaling | 3.25 |
| GABA Receptor Signaling | 3.08 |
| nNOS Signaling in Skeletal Muscle Cells | 3.08 |
| *Pathways with -log(p-value) $\geq$ 1.3 are reported here as statistically significant | |

**Shared DNA methylation pathways and CPSP and CASI**

Significant CpG sites associated with CPSP were annotated to 310 genes, and those for CASI were located on 1526 genes. At the gene level, 39 genes had CpG sites with significant DNA methylation associations with both outcomes, and they mapped to 14 pathways. Shared overlapping pathways identified by gene overlap for CpG sites associated with both chronic post-surgical pain and child anxiety sensitivity index are detailed in Table 5. The top pathways were GABA receptor signaling and Dopamine-DARPP32 Feedback in cAMP Signaling (Figs. 3A-B).

Table 5: Shared overlapping pathways identified by gene overlap for CpG sites associated with both chronic post-surgical pain and child anxiety sensitivity index

| Shared Ingenuity Canonical Pathways for CPSP and CASI* | P-value CPSP | Ratio | Molecules | P-value CASI | Ratio |
|---|---|---|---|---|---|
| GABA Receptor Signaling | 0.00016 | 0.074 | ABAT,ADCY5,CACNA1H,CACNA1C,GABBR 1KCNH2, CACNA1A | 0.0008 | 0.158 |
| Dopamine-DARPP32 Feedback in cAMP Signaling | 0.004 | 0.043 | PPP1R1B, ADCY5, PLCG2, CAMKK1, CACNA1C,DRD4, CACNA1A | 0.00003 | 0.152 |
| Cellular Effects of Sildenafil (Viagra) | 0.005 | 0.046 | ADCY5, PLCG2, CACNA1C, NPPA, KCNH2, CACNA1A | 0.0002 | 0.153 |
| GPCR-Mediated Nutrient Sensing in Enteroendocrine Cells | 0.012 | 0.045 | ADCY5,PLCG2,CACNA1H,CACNA1C,CACN A1A | 0.0002 | 0.161 |
| Calcium Signaling | 0.013 | 0.034 | CAMKK1, TRDN, CACNA1H, CACNA1C, CACNA1A, ATP2B2, CAMK2B | 0.0026 | 0.117 |
| nNOS Signaling in Skeletal Muscle Cells | 0.013 | 0.073 | CACNA1H, CACNA1C, CACNA1A | 0.0008 | 0.220 |

(continued)

| Shared Ingenuity Canonical Pathways for CPSP and CASI* | P-value CPSP | Ratio | Molecules | P-value CASI | Ratio |
|---|---|---|---|---|---|
| Dopamine Receptor Signaling | 0.015 | 0.052 | *PPP1R1B, ADCY5, DRD4, SLC18A2* | 0.00007 | 0.195 |
| Synaptic Long Term Depression | 0.020 | 0.035 | *PLBD1,PLCG2,PLA2G4C,CACNA1H,CACNA1CCACNA1A* | 0.02570 | 0.103 |
| cAMP-mediated signaling | 0.022 | 0.031 | *PDE9A, ADCY5, VIPR2, PTHIR, GABBR1, DRD4, CAMK2B* | 0.000002 | 0.150 |
| Corticotropin Releasing Hormone Signaling | 0.028 | 0.036 | *ADCY5,PLCG2,CACNA1H,CACNA1C,CACNA1A* | 0.0064 | 0.122 |
| Netrin Signaling | 0.045 | 0.046 | *CACNA1H, CACNA1C, CACNA1A* | 0.0006 | 0.185 |
| CREB Signaling in Neurons | 0.046 | 0.028 | *ADCY5,PLCG2,CACNA1H,CACNA1C,CACNA1A, CAMK2B* | 0.0008 | 0.123 |
| *Two pathways not included in the table above include Gustatory pathway and sperm motility which are not relevant to the outcomes being studied | | | | | |

## Functional genomics analyses

Significantly methylated CpG sites associated with CPSP are located in active regulatory regions with open chromatin marked by H3K27ac, H3K4me1 and H3K4me3 in brain cells from the hippocampus, frontal lobe, temporal lobe, anterior cingulate cortex, etc. (Table 6A). Also depicted in Table 6A are the significant (p<0.05, after correction for multiple testing) protein (e.g., transcription factor) binding events identified to overlap significantly at the CpG sites, significant for CASI. Of note, many involve the RNA polymerase subunit POLR2A, suggesting that many differential methylation events might result in altered gene expression. Table 6B shows the genomic locations of the specific histone markers of the CpG sites associated with Chronic postsurgical pain (CPSP) and Table 6B-1 shows similar data for Childhood Anxiety Sensitivity Index (CASI). Table 6C shows the location of certain histone markers found in the regulatory regions of the indicated genes in multiple cell lines. Although we do not have expression data from the brain, the CpG sites depicted in Table 6C are located within brain sites associated with several active chromatin markers. For example, H3K27me3 ChIP-seq peaks in brain cells is a repressive (polycomb) signal. Finding these sites overlapping with similar markers from several brain sites important for nociception indicates that they are functional in those areas, and hence alter gene expression. In each of Tables 6B and 6C, the CpG sites are designated by their Illumina Identification number, "cg" followed by a number, and their position is relative to the human reference genome released February 27, 2009 by the Genome Reference Consortium (GRC) referred to as GRCh37 or HG19.

Table 6A: Overlap between CpG sites associated with chronic postsurgical pain (CPSP) and childhood anxiety sensitivity index (CASI) with functional genomics datasets in cells derived from brain tissue

| CpG sites associated with Chronic postsurgical pain (Histone markers) | | | | |
|---|---|---|---|---|
| Dataset | Cell type | Epigenetic mark | Ratio | Corrected P-value |
| Roadmap Epigenomics (Histone narrow) | Brain (Hippocampus, Middle) | H3K27me3 | 0.226 | 1.37E-14 |
| Roadmap Epigenomics (Histone narrow) | Brain (Mid Frontal Lobe) | H3K27me3 | 0.190 | 3.56E-13 |
| Roadmap Epigenomics (Histone narrow) | Fetal (Brain, Male) | H3K27me3 | 0.187 | 1.96E-09 |
| Roadmap Epigenomics (Histone narrow) | Brain (Inferior Temporal Lobe) | H3K27me3 | 0.154 | 1.11E-07 |

(continued)

| CpG sites associated with Chronic postsurgical pain (Histone markers) | | | | |
|---|---|---|---|---|
| **Dataset** | **Cell type** | **Epigenetic mark** | **Ratio** | **Corrected P-value** |
| Roadmap Epigenomics (Histone narrow) | Brain (Cingulate Gyrus) | H3K27me3 | 0.146 | 5.68E-07 |
| Roadmap Epigenomics (Histone narrow) | Fetal (Brain, Male) | H3K4me1 | 0.349 | 6.03E-07 |
| Roadmap Epigenomics (Histone narrow) | Fetal (Brain, Female) | H3K27me3 | 0.185 | 8.03E-06 |
| Roadmap Epigenomics (Active Chromatin) | Brain (Germinal Matrix) | Bivalent enhancer | 0.046 | 9.36E-05 |
| Roadmap Epigenomics (Histone narrow) | Brain (Substantia Nigra) | H3K27me3 | 0.119 | 0.00014 |
| Roadmap Epigenomics (Histone narrow) | Brain (Angular Gyrus) | H3K27me3 | 0.127 | 0.0015 |
| Roadmap Epigenomics (Active Chromatin) | Fetal (Brain, Male) | Bivalent enhancer | 0.096 | 0.0055 |
| Roadmap Epigenomics (Active Chromatin) | Fetal (Brain, Male) | Bivalent TSS | 0.019 | 0.0109 |
| eQTLs (GTEx V6) | Brain (Anterior cingulate cortex BA24) | eQTL | 0.025 | 0.015 |
| CpG sites associated with Childhood anxiety Sensitivity Index (Histone markers) | | | | |
| Roadmap Epigenomics (Histone narrow) | Brain (Cingulate Gyrus) | H3K4me3 | 0.321 | 6.76E-10 |
| Roadmap Epigenomics (Histone narrow) | Brain (Mid Frontal Lobe) | H3K4me3 | 0.321 | 8.98E-09 |
| Roadmap Epigenomics (Dnase narrow) | H1 Derived Neuronal Progenitor Cells | Dnase | 0.381 | 1.37E-08 |
| Roadmap Epigenomics (Histone narrow) | Brain (Inferior Temporal Lobe) | H3K4me3 | 0.321 | 2.26E-07 |
| Roadmap Epigenomics (Histone narrow) | H9_Derived_Neuron_Cultured_Cells | H2A.Z | 0.251 | 4.26E-07 |
| Roadmap Epigenomics (Histone narrow) | H9 Derived Neuronal Progenitor Cells | H2A.Z | 0.334 | 5.73E-07 |
| Roadmap Epigenomics (Histone narrow) | Brain (Hippocampus Middle) | H3K4me3 | 0.333 | 7.51E-07 |
| Roadmap Epigenomics (Histone narrow) | Brain (Anterior Caudate) | H3K4me3 | 0.330 | 1.09E-06 |
| Roadmap Epigenomics (Histone narrow) | Brain (Angular Gyrus) | H3K4me3 | 0.299 | 1.49E-06 |
| Roadmap Epigenomics (Histone narrow) | Brain (Substantia Nigra) | H3K4me3 | 0.294 | 2.00E-06 |
| Roadmap Epigenomics (Histone narrow) | H9_Derived_Neuron_Cultured_Cells | H3K4me3 | 0.256 | 1.11E-05 |
| Roadmap Epigenomics (Histone narrow) | H9 Derived Neuronal Progenitor Cells | H3K4me3 | 0.250 | 3.48E-05 |
| Roadmap Epigenomics (Active Chromatin) | Brain (Hippocampus Middle) | 2_TssAFlnk | 0.103 | 0.0001 |

(continued)

| CpG sites associated with Childhood anxiety Sensitivity Index (Histone markers) | | | | |
|---|---|---|---|---|
| Roadmap Epigenomics (Histone narrow) | H1 Derived Neuronal Progenitor Cells | H3K4me2 | 0.283 | 0.0002 |
| Roadmap Epigenomics (Active Chromatin) | Brain (Anterior Caudate) | 1_TssA | 0.232 | 0.0006 |
| DNaseI Duke | Cerebellum | Dnase | 0.314 | 0.0007 |
| Roadmap Epigenomics (Active Chromatin) | Brain (Cingulate Gyrus) | ActiveChromatin | 0.381 | 0.0007 |
| Roadmap Epigenomics (Active Chromatin) | Brain (Inferior Temporal Lobe) | ActiveChromatin | 0.383 | 0.0009 |
| Roadmap Epigenomics (Active Chromatin) | Brain (Inferior Temporal Lobe) | 2_TssAFlnk | 0.091 | 0.0010 |
| Roadmap Epigenomics (Active Chromatin) | H9_Derived_Neuron | ActiveChromatin | 0.356 | 0.0014 |
| CpG sites associated with Chronic postsurgical pain (Histone markers) | | | | |
| **Dataset** | **Cell type** | **Epigenetic mark** | **Ratio** | **Corrected P-value** |
| Roadmap Epigenomics (Active Chromatin) | Brain (Substantia Nigra) | 1_TssA | 0.211 | 0.0019 |
| Roadmap Epigenomics (Active Chromatin) | Brain (Anterior Caudate) | ActiveChromatin | 0.391 | 0.0022 |
| Roadmap Epigenomics (Active Chromatin) | H1_Derived_Neuronal_Progenitor | ActiveChromatin | 0.317 | 0.0024 |
| Roadmap Epigenomics (Active Chromatin) | Brain (Angular Gyrus) | 1_TssA | 0.220 | 0.0028 |
| Roadmap Epigenomics (Histone narrow) | Brain (Substantia Nigra) | H3K9ac | 0.264 | 0.0052 |
| Roadmap Epigenomics (Active Chromatin) | Brain (Hippocampus Middle) | 1_TssA | 0.201 | 0.0078 |
| Roadmap Epigenomics (Active Chromatin) | Brain (Substantia Nigra) | ActiveChromatin | 0.363 | 0.0085 |
| Roadmap Epigenomics (Active Chromatin) | Brain (Cingulate Gyrus) | 2_TssAFlnk | 0.088 | 0.0112 |
| Roadmap Epigenomics (Active Chromatin) | Brain (Cingulate Gyrus) | 1_TssA | 0.203 | 0.0116 |
| Roadmap Epigenomics (Histone narrow) | Brain (Anterior Caudate) | H3K9ac | 0.302 | 0.0227 |
| DNaseI Duke | Frontal cortex | Dnase | 0.386 | 0.0232 |
| Roadmap Epigenomics (Histone narrow) | Brain (Mid Frontal Lobe) | H3K9ac | 0.267 | 0.0246 |
| Roadmap Epigenomics (Histone narrow) | Brain (Inferior Temporal Lobe) | H3K9ac | 0.310 | 0.0279 |
| Roadmap Epigenomics (Histone narrow) | Brain (Cingulate Gyrus) | H3K9ac | 0.294 | 0.0314 |
| Roadmap Epigenomics (Active Chromatin) | H9_Derived_Neuronal_Progenitor | ActiveChromatin | 0.340 | 0.0329 |

(continued)

| CpG sites associated with Chronic postsurgical pain (Histone markers) | | | | |
|---|---|---|---|---|
| Dataset | Cell type | Epigenetic mark | Ratio | Corrected P-value |
| Roadmap Epigenomics (Active Chromatin) | Brain (Hippocampus Middle) | ActiveChromatin | 0.402 | 0.0395 |
| Roadmap Epigenomics (Active Chromatin) | Brain (Angular Gyrus) | 2_TssAFlnk | 0.069 | 0.0478 |
| CpG sites associated with Childhood anxiety Sensitivity Index (Transcription factors) | | | | |
| Dataset | Cell type | Protein | Ratio | Corrected P-value |
| ENCODE | HepG2+forskolin | POLR2A | 0.175 | 0.00032 |
| ENCODE | MCF10A-Er-Src+4OHTAM_1uM_36hr | POLR2A | 0.169 | 0.00053 |
| ENCODE | MCF10A-Er-Src+EtOH_0.01pct | POLR2A | 0.171 | 0.00058 |
| Cistrome | HEK293 | BRD2 | 0.209 | 0.0012 |
| ENCODE | MCF-7 | POLR2A | 0.155 | 0.0013 |
| ENCODE | MCF-7+serum_stimulated_media | POLR2A | 0.172 | 0.0017 |
| Cistrome | CUTLL1 | ETS1 | 0.169 | 0.0028 |
| ENCODE | ProgFib | POLR2A | 0.160 | 0.0044 |
| ENCODE | HCT-116 | POLR2A | 0.190 | 0.0047 |
| ENCODE | HeLa-S3 | POLR2A | 0.186 | 0.0068 |
| ENCODE | Gliobla | POLR2A | 0.165 | 0.0078 |
| ENCODE | A549+DEX_100nM | POLR2A | 0.191 | 0.0078 |
| ENCODE | A549+EtOH_0.02pct | POLR2A | 0.191 | 0.0082 |
| ENCODE | ECC-1+DMSO_0.02pct | POLR2A | 0.174 | 0.0093 |
| ENCODE | MCF-7+serum_starved_media | POLR2A | 0.167 | 0.0093 |
| ENCODE | NB4 | POLR2A | 0.156 | 0.011 |
| ENCODE | H1-hESC | POLR2A | 0.173 | 0.013 |
| ReMap | hek293 | BRD3 | 0.077 | 0.014 |
| ENCODE | A549 | POLR2A | 0.169 | 0.014 |
| ENCODE | H1-HESC | RBBP5 | 0.112 | 0.014 |
| ENCODE | HUVEC | POLR2A | 0.198 | 0.016 |
| Misc (GEO) | LoVo | ASCL2 | 0.124 | 0.020 |
| ENCODE | SK-N-MC | POLR2A | 0.137 | 0.020 |
| ENCODE | GM19099 | POLR2A | 0.170 | 0.023 |
| ENCODE | GM15510 | POLR2A | 0.165 | 0.025 |
| ENCODE | MCF-7+serum_starved_media | CTCF | 0.113 | 0.026 |
| ENCODE | GM12878 | POLR2A | 0.188 | 0.034 |
| Misc (GEO) | LoVo | GMEB2 | 0.166 | 0.035 |
| Pazar | Co4+ | HMGN1 | 0.198 | 0.039 |
| Misc (GEO) | LoVo | MEF2C | 0.039 | 0.039 |

(continued)

| CpG sites associated with Childhood anxiety Sensitivity Index (Transcription factors) | | | | |
|---|---|---|---|---|
| **Dataset** | **Cell type** | **Protein** | **Ratio** | **Corrected P-value** |
| Misc (GEO) | LoVo | RAD21 | 0.261 | 0.044 |
| 'Ratio' indicates the fraction of CPSP or CASI differentially methylated CpGs whose genomic coordinates intersect the indicated dataset. P-value is based on the significance of the ratio, and is adjusted for multiple testing, based on simulations (see Methods). No significant transcription factors in brain cells were identified for CpG sites associated with CPSP. | | | | |

Table 6B: Overlap of CpG sites associated with Chronic postsurgical pain (Histone markers)

| Track | Cell | TF | Overlap | CpG Sitegenom | P-val | Corrected P-val |
|---|---|---|---|---|---|---|
| Roadmapepigenomics_ Histone_narrow | Brain_Hi ppocam pus_Mid dle | H3K2 7me3 | 144 | cg00098609,cg00106345,cg00292513,cg00470007,cg00840694,cg010217 42,cg01446203,cg01582077,cg01601949,cg01700504,cg01749904,cg0182 0273,cg02104434,cg02273436,cg02305659,cg02399249,cg02518691,cg02 575092,cg02578584,cg02579903,cg02675264,cg02920396,cg02931642,cg 02978505,cg03139896,cg03188390,cg03382370,cg03791425,cg03888083, cg04497389,cg04913730,cg05059566,cg05606455,cg05780180,cg058252 44,cg05888755,cg06101084,cg06662252,cg07119157,cg07192748,cg0721 2818,cg07781491,cg07788486,cg07832006,cg07895523,cg08087969,cg08 121925,cg08133631,cg08150668,cg08421459,cg08643994,cg08856033,cg 08992249,cg09015880,cg09066676,cg09652503,cg09826364,cg10131124, cg10403784,cg10583942,cg10694470,cg10695647,cg10735475,cg107475 31,cg10906729,cg11024728,cg11205636,cg11211795,cg11234688,cg1133 4475,cg11359720,cg11479811,cg11480627,cg11988807,cg12284098,cg12 751644,cg12754571,cg13152974,cg13546858,cg13579486,cg13608716,cg 13938349,cg14042396,cg14161269,cg14329049,cg14773235,cg15191795, cg15417294,cg15572235,cg15691140,cg15981071,cg16000637,cg160171 44,cg16050468,cg16065768,cg16227623,cg16312002,cg16350349,cg1746 3745,cg17545182,cg17655970,cg17733447,cg17932010,cg18988382,cg19 059839,cg19409579,cg19628603,cg19990135,cg20561938,cg21377071,cg 21551271,cg21606928,cg21746969,cg21822583,cg22234712,cg22534288, cg22682200,cg22830507,cg23201527,cg23240888,cg23329272,cg238279 50,cg23942884,cg24211006,cg24362661,cg24375409,cg24446178,cg2469 4501,cg25129124,cg25497175,cg25665636,cg25943719,cg26186954,cg26 299084,cg26303165,cg26305042,cg26843498,cg26864301,cg26995942,cg 27018912,cg27179866,cg27300742,cg27570304,cg27625507 | 5.48E-18 | 1.37E-14 |
| Roadmapepigenomics_ Histone_narrow | Brain_M id_Front al_Lobe | H3K2 7me3 | 121 | cg00098609,cg00106345,cg00292513,cg00470007,cg00840694,cg014303 44,cg01446203,cg01582077,cg01601949,cg01700504,cg01749904,cg0182 0273,cg02273436,cg02399249,cg02518691,cg02579903,cg02675264,cg02 931642,cg03139896,cg03188390,cg03382370,cg03469862,cg03791425,cg 03966315,cg04913730,cg05029551,cg05059566,cg05074213,cg05888755, cg06019884,cg06307913,cg06662252,cg07119157,cg07212818,cg077814 91,cg07788486,cg07832006,cg07895523,cg08087969,cg08150668,cg0885 6033,cg08983883,cg08992249,cg09066676,cg09652503,cg09745633,cg09 | 1.42E-16 | 3.56E-13 |

EP 3 813 814 B1

| Track | Cell | TF | Overlap | CpG Sitegenom | P-val | Corrected P-val |
|---|---|---|---|---|---|---|
| | | | | 826364,cg10131124,cg10478584,cg10583942,cg10694470,cg10735475,cg10747531,cg10906729,cg11024728,cg11211795,cg11234688,cg11359720,cg11988807,cg12236088,cg12298207,cg13060282,cg13546858,cg13579486,cg13872005,cg14024810,cg14042396,cg14161269,cg14329049,cg14773235,cg15191795,cg15234197,cg15417294,cg15572235,cg15671450,cg15691140,cg15735453,cg15981071,cg16000637,cg16050468,cg16065768,cg16227623,cg16312002,cg17463745,cg17655970,cg17681294,cg17733447,cg18073471,cg19059839,cg19409579,cg20506843,cg21320242,cg21377071,cg21746969,cg21822583,cg22157099,cg22234712,cg22534288,cg22641072,cg22682200,cg22902505,cg23240888,cg23549902,cg24211006,cg24362661,cg24375409,cg24446178,cg24694501,cg24707404,cg24838316,cg25428553,cg25665636,cg26299084,cg26843498,cg26995942,cg27008027,cg27018912,cg27242132,cg27485108,cg27570304,cg27625507 | | |
| Roadmapepigenomics_Histone_narrow | Fetal_Brain_Male | H3K27me3 | 119 | cg00106345,cg00292513,cg00579921,cg01385412,cg01446203,cg01700504,cg01708427,cg01820273,cg01899676,cg02104434,cg02399249,cg02518691,cg02575092,cg02579903,cg02675264,cg02920396,cg02931642,cg03139896,cg03382370,cg03469862,cg03791425,cg03888083,cg04497389,cg04538261,cg04913730,cg05537355,cg05780180,cg05825244,cg05888755,cg06019884,cg06178828,cg06188548,cg06503981,cg06662252,cg06699671,cg07119157,cg07212818,cg07781491,cg07832006,cg07882059,cg08310581,cg08643994,cg08748308,cg08983883,cg09015880,cg09652503,cg10403784,cg10478584,cg10583942,cg10695647,cg10735475,cg10747531,cg10906729,cg11211795,cg11359720,cg11748187,cg11988807,cg12222949,cg12297282,cg12751644,cg13152974,cg13546858,cg13579486,cg13608716,cg13938349,cg14024810,cg14161269,cg14329049,cg15234197,cg15691140,cg15793688,cg15981071,cg16000637,cg16065768,cg16227623,cg16312002,cg16350349,cg16702083,cg17463745,cg17655970,cg17733447,cg17870959,cg19059839,cg19628603,cg20561938,cg20597747,cg20794334,cg21341928,cg21377071,cg21499289,cg21551271,cg21822583,cg22128410,cg22234712,cg22682200,cg22830507,cg23240888,cg23329272,cg23365293,cg23740940,cg23942884,cg24211006,cg24362661,cg24375409,cg24524379,cg24838316,cg24947451,cg25129124,cg25454379,cg25665636,cg25943719,cg26186954,cg26843498,cg26899284,cg27179866,cg27454650,cg27485108,cg27570304,cg27625507 | 7.83E-13 | 1.96E-09 |
| Roadmapepigenomics_Histone_narrow | Brain_Inferior_T | H3K27me3 | 98 | cg00106345,cg00292513,cg01446203,cg01700504,cg01708427,cg01749904,cg01820273,cg02113641,cg02273436,cg02675264,cg02931642,cg0297 | 4.43E-11 | 1.11E-07 |

| Track | Cell | TF | Overlap | CpG Sitegenom | P-val | Corrected P-val |
|---|---|---|---|---|---|---|
| | emporal_Lobe | | | 8505,cg03139896,cg03188390,cg03382370,cg03791425,cg04505435,cg04538261,cg04913730,cg05888755,cg06188548,cg06662252,cg07212818,cg07542748,cg07781491,cg07832006,cg07895523,cg08087969,cg08643994,cg08767820,cg08856033,cg08983883,cg08992249,cg09015880,cg09633081,cg10403784,cg10694470,cg10695647,cg10735475,cg10906729,cg11024728,cg11211795,cg11334475,cg11480627,cg11988807,cg12284098,cg13546858,cg13579486,cg13608716,cg14024810,cg14161269,cg14329049,cg14773235,cg15124926,cg15191795,cg15417294,cg16000637,cg16065768,cg16134678,cg16227623,cg16312002,cg16350349,cg17463745,cg17655970,cg17733447,cg17870959,cg18073471,cg18583910,cg19059839,cg19409579,cg19628603,cg20561938,cg21341928,cg21377071,cg21480165,cg21822583,cg22234712,cg22682200,cg22830507,cg22902505,cg23201527,cg23329272,cg23942884,cg24211006,cg24362661,cg24446178,cg25129124,cg25454379,cg25497175,cg25665636,cg26186954,cg26299084,cg26843498,cg27018912,cg27179866,cg27242132,cg27485108,cg27625507 | | |
| Roadmapepigenomics_Histone_narrow | Brain_Cingulate_Gyrus | H3K27me3 | 93 | cg00106345,cg01446203,cg01700504,cg01820273,cg02273436,cg02399249,cg02675264,cg02920396,cg02931642,cg03139896,cg03188390,cg03382370,cg03791425,cg03966315,cg04538261,cg04913730,cg05059566,cg05780180,cg05888755,cg07119157,cg07192748,cg07212818,cg07832006,cg07895523,cg08087969,cg08150668,cg08643994,cg08983883,cg08992249,cg09066676,cg09633081,cg09652503,cg10131124,cg10403784,cg10583942,cg10694470,cg10735475,cg10747531,cg10906729,cg10923851,cg11024728,cg11359720,cg11988807,cg12297282,cg13546858,cg13579486,cg13608716,cg13933849,cg14024810,cg14161269,cg14773235,cg15094605,cg15124926,cg15191795,cg15255086,cg15417294,cg15557760,cg15691140,cg15981071,cg16000637,cg16065768,cg16227623,cg16312002,cg16350349,cg17463745,cg17655970,cg17733447,cg17932010,cg18073471,cg19059839,cg19409579,cg20561938,cg21377071,cg21499289,cg21822583,cg22234712,cg22682200,cg22902505,cg23201527,cg23240888,cg24211006,cg24356544,cg24362661,cg24446178,cg25665636,cg25863732,cg25943719,cg26299084,cg26843498,cg27018912,cg27242132,cg27570304,cg27625507 | 2.27E-10 | 5.68E-07 |
| Roadmapepigenomics_Histone_narrow | Fetal_Brain_Male | H3K4me1 | 222 | cg00106345,cg00292513,cg00579921,cg01385412,cg01446203,cg01700504,cg01708427,cg01820273,cg01899676,cg02104434,cg02399249,cg02518691,cg02575092,cg02579903,cg02675264,cg02920396,cg02931642,cg03139896,cg03382370,cg03469862,cg03791425,cg03888083,cg04497389,cg | 2.41E-10 | 6.03E-07 |

| Track | Cell | TF | Overlap | CpG Sitegenom | P-val | Corrected P-val |
|---|---|---|---|---|---|---|
| | | | | 04538261,cg04913730,cg05537355,cg05780180,cg05825244,cg05888755, cg06019884,cg06178828,cg06188548,cg06503981,cg06662252,cg066996 71,cg07119157,cg07212818,cg07781491,cg07832006,cg07882059,cg0831 0581,cg08643994,cg08748308,cg08983883,cg09015880,cg09652503,cg10 403784,cg10478584,cg10583942,cg10695647,cg10735475,cg10747531,cg 10906729,cg11211795,cg11359720,cg11748187,cg11988807,cg12222949, cg12297282,cg12751644,cg13152974,cg13546858,cg13579486,cg136087 16,cg13938349,cg14024810,cg14161269,cg14329049,cg15234197,cg1569 1140,cg15793688,cg15981071,cg16000637,cg16065768,cg16227623,cg16 312002,cg16350349,cg16702083,cg17463745,cg17655970,cg17733447,cg 17870959,cg19059839,cg19628603,cg20561938,cg20597747,cg20794334, cg21341928,cg21377071,cg21499289,cg21551271,cg21822583,cg221284 10,cg22234712,cg22682200,cg22830507,cg23240888,cg23329272,cg2336 5293,cg23740940,cg23942884,cg24211006,cg24362661,cg24375409,cg24 524379,cg24838316,cg24947451,cg25129124,cg25454379,cg25665636,cg 25943719,cg26186954,cg26843498,cg26899284,cg27179866,cg27454650, cg27485108,cg27570304,cg27625507 | | |
| Roadmapepigenomics_ Histone_narrow | Fetal_Br ain_Fem ale | H3K2 7me3 | 118 | cg00106345,cg00292513,cg01446203,cg01456368,cg01700504,cg017084 27,cg01820273,cg01899676,cg02113641,cg02399249,cg02575092,cg0263 0677,cg02675264,cg02920396,cg02931642,cg03139896,cg03382370,cg03 791425,cg03888083,cg04497389,cg04521867,cg04538261,cg04913730,cg 05780180,cg05825244,cg05888755,cg06019884,cg06178828,cg06307913, cg06662252,cg06699671,cg06905124,cg07119157,cg07212818,cg077814 91,cg07788486,cg07832006,cg08087969,cg08133631,cg08310581,cg0864 3994,cg08699441,cg08983883,cg09015880,cg09652503,cg09826364,cg10 403784,cg10478584,cg10583942,cg10695647,cg10735475,cg10747531,cg 10906729,cg11211795,cg11359720,cg11748187,cg11988807,cg12222949, cg12236088,cg13546858,cg13579486,cg13608716,cg13938349,cg141612 69,cg14329049,cg14606549,cg15981071,cg16000637,cg16065768,cg1613 4678,cg16227623,cg16312002,cg16323913,cg16350349,cg17084373,cg17 463745,cg17655970,cg17733447,cg18073471,cg18583910,cg19059839,cg 19628603,cg20561938,cg20794334,cg21320242,cg21341928,cg21377071, cg21480165,cg21499289,cg21551271,cg21822583,cg22128410,cg222347 12,cg22360016,cg22682200,cg22684266,cg22830507,cg22902505,cg2324 0888,cg23329272,cg23942884,cg24211006,cg24356544,cg24362661,cg24 375409,cg24947451,cg25497175,cg25665636,cg25943719,cg26186954,cg | 3.21E-09 | 8.03E-06 |

| Track | Cell | TF | Overlap | CpG Sitegenom | P-val | Corrected P-val |
|---|---|---|---|---|---|---|
| | | | | 26843498,cg26899284,cg27179866,cg27242132,cg27300742,cg27485108, cg27570304,cg27625507 | | |
| Roadmapepigenomics_ ActiveChromatin | Brain_G erminal_ Matrix | 12_E nhBiv | 29 | cg00292513,cg01446203,cg02399249,cg02518691,cg02931642,cg031398 96,cg05825244,cg07212818,cg07781491,cg07832006,cg08643994,cg0916 4913,cg10735475,cg10747531,cg11211795,cg13579486,cg14024810,cg16 017144,cg16312002,cg17463745,cg17733447,cg20561938,cg21320242,cg 21377071,cg22243109,cg23329272,cg24211006,cg24362661,cg26186954 | 3.74E-08 | 9.36E-05 |
| Roadmapepigenomics_ Histone_narrow | Brain_Su bstantia _Nigra | H3K2 7me3 | 76 | cg00098609,cg00106345,cg00470007,cg01446203,cg01700504,cg017499 04,cg01820273,cg02518691,cg02978505,cg03010138,cg03188390,cg0338 2370,cg04414307,cg04505435,cg04913730,cg05059566,cg05155922,cg05 780180,cg05888755,cg06188548,cg07119157,cg07832006,cg07895523,cg 08121925,cg08150668,cg08983883,cg08992249,cg09015880,cg10583942, cg10694470,cg10747531,cg10906729,cg11024728,cg11294620,cg113597 20,cg11480627,cg11988807,cg13060282,cg13938349,cg14024810,cg1416 1269,cg14773235,cg15417294,cg15691140,cg15981071,cg16000637,cg16 050468,cg16065768,cg16350349,cg17463745,cg17545182,cg17655970,cg 17733447,cg17932010,cg18988382,cg19059839,cg19409579,cg19418809, cg20023621,cg20561938,cg21822583,cg22128410,cg22682200,cg228305 07,cg23329272,cg24211006,cg24362661,cg24375409,cg25665636,cg2618 6954,cg26299084,cg26843498,cg26995942,cg27018912,cg27570304,cg27 625507 | 5.66E-08 | 0.000142 |
| Roadmapepigenomics_ Histone_narrow | Brain_A ngular_ Gyrus | H3K2 7me3 | 81 | cg00106345,cg01446203,cg01700504,cg01749904,cg01820273,cg021044 34,cg02273436,cg02399249,cg02579903,cg02675264,cg02931642,cg0313 9896,cg03382370,cg03966315,cg04913730,cg05074213,cg05780180,cg05 888755,cg06188548,cg06307913,cg06662252,cg07119157,cg07192748,cg 07212818,cg07781491,cg07788486,cg07832006,cg08087969,cg08121925, cg08643994,cg08983883,cg09015880,cg09066676,cg09633081,cg096525 03,cg10131124,cg10213542,cg10403784,cg10583942,cg10735475,cg1074 7531,cg10906729,cg11024728,cg11211795,cg11988807,cg12751644,cg12 754571,cg13152974,cg13507983,cg13579486,cg14329049,cg14773235,cg 15417294,cg15691140,cg16000637,cg16065768,cg16227623,cg16312002, cg16350349,cg17655970,cg17733447,cg18073471,cg19059839,cg194188 09,cg20561938,cg21377071,cg21499289,cg21822583,cg22234712,cg2268 2200,cg22830507,cg22902505,cg23240888,cg23329272,cg24211006,cg24 362661,cg24707404,cg25454379,cg25665636,cg26843498,cg27242132 | 6.14E-07 | 0.001536 |

| Track | Cell | TF | Overlap | CpG Sitegenom | P-val | Corrected P-val |
|---|---|---|---|---|---|---|
| Roadmapepigenomics_ActiveChromatin | Fetal_Brain_Male | 12_EnhBiv | 61 | cg00292513,cg00579921,cg01446203,cg01456368,cg01899676,cg0239924 9,cg02518691,cg02775617,cg02931642,cg03139896,cg03469862,cg0388 8083,cg04497389,cg05780180,cg05825244,cg06699671,cg07212818,cg07 425885,cg07781491,cg07882059,cg08121925,cg08310581,cg08643994,cg 08748308,cg08856033,cg09652503,cg10403784,cg10735475,cg10747531, cg11748187,cg12222949,cg12297282,cg13872005,cg13938349,cg140248 10,cg15234197,cg16065768,cg16312002,cg16350349,cg17463745,cg1765 5970,cg20291162,cg20794334,cg21320242,cg21377071,cg21499289,cg21 551271,cg21822583,cg22128410,cg22234712,cg23201527,cg23240888,cg 23329272,cg23365293,cg23740940,cg24211006,cg24362661,cg24524379, cg25129124,cg26186954,cg27485108 | 2.20E-06 | 0.005505 |
| Roadmapepigenomics_ActiveChromatin | Fetal_Brain_Male | 10_TssBiv | 12 | cg00106345,cg05059566,cg06188548,cg06307913,cg10694470,cg180734 71,cg19409579,cg22682200,cg22902505,cg26299084,cg27018912,cg2724 2132 | 4.38E-06 | 0.010958 |
| eQTLs_GTEx_Analysis_V6 | Brain_Anterior_cingulate_cortex_BA24 | none | 16 | cg00579921,cg03810198,cg05546241,cg06503981,cg06507124,cg081336 31,cg08431931,cg13025000,cg15671450,cg17763019,cg19059839,cg1958 5676,cg23303505,cg23365293,cg24838316,cg26739327 | 6.18E-06 | 0.015469 |

24

EP 3 813 814 B1

Table 6B-1: Overlap of CpG sites associated with Childhood Anxiety Sensitivity Index

| Track | Cell | TF | Overlap | | P-val | Correct ed P-val |
|---|---|---|---|---|---|---|
| TxnFactor ChIPV3 | Globla | POLR2A | 403 | cg00021028,cg00028135,cg00106446,cg00347856,cg00556087, cg00571506,cg00603625,cg00644777,cg00670721,cg00702057, cg00724098,cg00855501,cg00862770,cg00874835,cg00877095, cg00916030,cg01023668,cg01160766,cg01232495,cg01307507, cg01353677,cg01517384,cg01652542,cg01678753,cg01694696, | 5.02E-06 | 0.007 |

cg01796184,cg01862172,cg01877301,cg01959848,cg01960259,
cg02091366,cg02222250,cg02337960,cg02384115,cg02388253,
cg02732508,cg02766391,cg02835499,cg02962558,cg03183257,
cg03218003,cg03343262,cg03419014,cg03519303,cg03553358,
cg03580256,cg03593280,cg03639557,cg03640766,cg03657766,
cg03726236,cg03730309,cg03833068,cg03890362,cg03969906,
cg04011182,cg04209315,cg04219510,cg04221225,cg04278353,
cg04282206,cg04332235,cg04456228,cg04477202,cg04614053,
cg04620291,cg04638200,cg04807246,cg04865442,cg04999502,
cg05056497,cg05079543,cg05087948,cg05133900,cg05156137,
cg05171487,cg05173517,cg05272245,cg05334239,cg05668205,
cg05709321,cg05916509,cg06076054,cg06134546,cg06156157,
cg06185657,cg06277137,cg06296570,cg06405299,cg06446163,
cg06517138,cg06520003,cg06547771,cg06680147,cg06688411,
cg06712410,cg06925236,cg06992027,cg07160630,cg07319722,
cg07393878,cg07395907,cg07455279,cg07456797,cg07460645,
cg07504780,cg07532839,cg07595909,cg07679025,cg07785717,
cg07860992,cg08016528,cg08197201,cg08356572,cg08375775,
cg08410921,cg08433272,cg08480449,cg08578313,cg08583973,
cg08667096,cg08695336,cg08695707,cg08712717,cg08790485,
cg08949339,cg08964912,cg09175873,cg09552761,cg09592487,
cg09740319,cg09762316,cg09764697,cg09797645,cg09909833,
cg09910464,cg10011239,cg10189135,cg10201402,cg10231742,
cg10252181,cg10423031,cg10548983,cg10640944,cg10697117,
cg10784030,cg10878312,cg10885940,cg10893370,cg11033709,
cg11065244,cg11123972,cg11137517,cg11151820,cg11214243,
cg11318307,cg11487037,cg11520509,cg11579693,cg11606796,
cg11607276,cg11660684,cg11673280,cg11685814,cg11700824,
cg12023318,cg12148387,cg12218249,cg12394426,cg12396629,
cg12457901,cg12509004,cg12635877,cg12677248,cg12715979,
cg12761825,cg12793879,cg12798992,cg12844142,cg12873262,
cg12896170,cg12916174,cg12946660,cg13087771,cg13198594,
cg13258606,cg13264311,cg13268663,cg13402779,cg13468857,
cg13528349,cg13539030,cg13605142,cg13721560,cg13730193,
cg13781510,cg13790879,cg13841836,cg13850234,cg13933409,
cg13966771,cg14006390,cg14097294,cg14175438,cg14184873,

cg14195377,cg14240326,cg14332079,cg14345524,cg14401372,
cg14445518,cg14454588,cg14489649,cg14559336,cg14640751,
cg14656297,cg14752603,cg14798390,cg15073161,cg15126363,
cg15133540,cg15171982,cg15237047,cg15253648,cg15279002,
cg15497960,cg15513671,cg15716185,cg15821620,cg15848685,
cg15906799,cg16009787,cg16092834,cg16208271,cg16249821,
cg16341836,cg16487213,cg16511061,cg16557355,cg16628904,
cg16753670,cg16813552,cg16899367,cg16914123,cg16951854,
cg16958594,cg17074431,cg17189748,cg17290136,cg17477995,
cg17620457,cg17644557,cg17709718,cg17718488,cg17854078,
cg17864156,cg17886959,cg18049105,cg18090384,cg18121426,
cg18152809,cg18172804,cg18221121,cg18221850,cg18245083,
cg18364968,cg18374217,cg18483269,cg18506672,cg18512156,
cg18532190,cg18655928,cg18670564,cg18706544,cg18724257,
cg18885365,cg18912965,cg18942110,cg19100453,cg19135245,
cg19156220,cg19210358,cg19231821,cg19291606,cg19305511,
cg19508191,cg19612048,cg19627446,cg19679397,cg19716018,
cg19854901,cg19996355,cg20003603,cg20014596,cg20235885,
cg20253251,cg20276511,cg20447654,cg20706599,cg20710013,
cg20730280,cg20791505,cg20797740,cg20889476,cg20899437,
cg20909752,cg21161492,cg21195303,cg21196488,cg21205276,
cg21276217,cg21301258,cg21410231,cg21418052,cg21537108,
cg21575187,cg21739895,cg21840599,cg21868801,cg21915377,
cg22023257,cg22034155,cg22150978,cg22171725,cg22328901,
cg22387994,cg22499809,cg22508957,cg22532736,cg22560410,
cg22683308,cg22687206,cg22706166,cg22742943,cg22752023,
cg22771603,cg22935821,cg22941953,cg23100375,cg23127064,
cg23162904,cg23188378,cg23257934,cg23305229,cg23334729,
cg23361195,cg23411981,cg23681311,cg23779068,cg23783768,
cg23874008,cg23899092,cg23929381,cg23957084,cg24039393,
cg24119463,cg24166901,cg24249734,cg24319547,cg24321467,
cg24356473,cg24473277,cg24529996,cg24607545,cg24623422,
cg24633242,cg24662961,cg24744964,cg24818562,cg24841008,
cg24952075,cg25155022,cg25171089,cg25312873,cg25404375,
cg25419899,cg25485294,cg25503381,cg25548415,cg25602049,
cg25664725,cg25683204,cg25753555,cg25771615,cg26004235,

| TxnFactor ChIPV3 | SK-N-MC | POLR2A | 334 | cg00021028,cg00061233,cg00106446,cg00571506,cg00603625, cg00616582,cg00644777,cg00670721,cg00702057,cg00707134, cg00855501,cg00874835,cg00877095,cg00916030,cg01023668, cg01160766,cg01232495,cg01307507,cg01386775,cg01678753, cg01694696,cg01796184,cg01871533,cg01877301,cg01959848, cg02045085,cg02091366,cg02337960,cg02384115,cg02732508, cg02766391,cg02835499,cg03183257,cg03218003,cg03320372, cg03343262,cg03400443,cg03419014,cg03519303,cg03534684, cg03553358,cg03580256,cg03593280,cg03639557,cg03640766, cg03657766,cg03726236,cg03730309,cg03833068,cg03969906, cg04219510,cg04278353,cg04332235,cg04456228,cg04477202, cg04614053,cg04620291,cg04638200,cg04740205,cg04742985, cg04807246,cg04865442,cg04999502,cg05079543,cg05133900, cg05173517,cg05272245,cg05334239,cg05471845,cg05572047, cg05668205,cg05709321,cg05768702,cg06076054,cg06134546, cg06156157,cg06185657,cg06277137,cg06405299,cg06547771, cg06680147,cg06712410,cg06925236,cg06992027,cg07083785, cg07160630,cg07319722,cg07455279,cg07460645,cg07504780, cg07532839,cg07595909,cg07679025,cg07860992,cg08016528, cg08197201,cg08356572,cg08410921,cg08428677,cg08433272, cg08465120,cg08480449,cg08578313,cg08583973,cg08667096, cg08695707,cg08712717,cg08790485,cg09552761,cg09561351, cg09592487,cg09746391,cg09909833,cg09910464,cg10189135, cg10201402,cg10231742,cg10252181,cg10423031,cg10486424, cg10697117,cg10878312,cg10893370,cg11033709,cg11065244, cg11123972,cg11151820,cg11238542,cg11239695,cg11318307, cg11487037,cg11520509,cg11606796,cg11607276,cg11660684, cg11673280,cg11685814,cg12023318,cg12148387,cg12218249, cg12394426,cg12457901,cg12509004,cg12635877,cg12677248, | cg26112014,cg26117023,cg26147845,cg26227101,cg26269863, cg26376809,cg26397019,cg26678013,cg26912688,cg26995204, cg27079464,cg27109971,cg27206867,cg27265118,cg27544547, cg27581047,cg27665377, | 1.32E-05 | 0.020 |

28

cg12715979,cg12726807,cg12761825,cg12798992,cg12844142,
cg12873262,cg12896170,cg12916174,cg12946660,cg13087771,
cg13217116,cg13264311,cg13268663,cg13402779,cg13468857,
cg13676215,cg13721560,cg13730193,cg13781510,cg13788620,
cg13790879,cg13808314,cg13841836,cg13850234,cg13933409,
cg14006390,cg14175438,cg14184873,cg14195377,cg14240326,
cg14266862,cg14275340,cg14332079,cg14345524,cg14479751,
cg14489649,cg14559336,cg14640751,cg14656297,cg14752603,
cg14798390,cg15073161,cg15126363,cg15133540,cg15171982,
cg15173150,cg15253648,cg15279002,cg15513671,cg15587947,
cg15848685,cg15906799,cg16249821,cg16487213,cg16511061,
cg16628904,cg16750903,cg16813552,cg16899367,cg16951854,
cg16958594,cg16983211,cg17074431,cg17139210,cg17189748,
cg17208073,cg17224536,cg17290136,cg17477995,cg17620457,
cg17709718,cg17711960,cg17718488,cg17854078,cg17857086,
cg18152809,cg18172804,cg18221121,cg18221850,cg18245083,
cg18364968,cg18374217,cg18483269,cg18506672,cg18532190,
cg18617393,cg18634479,cg18655928,cg18706544,cg18724257,
cg18912965,cg18942110,cg19100453,cg19110902,cg19156220,
cg19231821,cg19291606,cg19305511,cg19427376,cg19508191,
cg19612048,cg19679397,cg19716018,cg19854901,cg19950474,
cg20235885,cg20253251,cg20706599,cg20730280,cg20737382,
cg20791505,cg20797740,cg20889476,cg20899437,cg20909752,
cg21161492,cg21195303,cg21205276,cg21276217,cg21418052,
cg21575187,cg21840599,cg21871091,cg22023257,cg22034155,
cg22150978,cg22171725,cg22328901,cg22499809,cg22508957,
cg22532736,cg22687206,cg22706166,cg22742943,cg22752023,
cg22771603,cg22935821,cg22941953,cg23127064,cg23162904,
cg23188378,cg23257934,cg23305229,cg23334729,cg23361195,
cg23681311,cg23779068,cg23874008,cg23929381,cg23957084,
cg24039393,cg24119463,cg24319547,cg24358762,cg24543015,
cg24607545,cg24633242,cg24744964,cg24818562,cg24841008,
cg25009842,cg25155022,cg25391820,cg25404375,cg25503381,
cg25602049,cg25664725,cg25683204,cg25695610,cg25705486,
cg26147845,cg26227101,cg26269863,cg26376809,cg26397019,
cg26479323,cg26498020,cg26678013,cg26912688,cg26995204,

cg27079464,cg27109971,cg27166037,cg27206867,cg27265118, cg27588119,cg27629640,cg27659974,cg27665377

Table 6C: Mapping of certain CpG sites to regulatory elements of genes

| IlmnID | Genome_Build | CHR | MAPINFO | UCSC_RefGene_Name |
|---|---|---|---|---|
| cg13060282 | 37 | 16 | 81882829 | PLCG2 |
| cg26995942 | 37 | 16 | 1226269 | CACNA1H; |
| cg21320242 | 37 | 17 | 37784895 | PPP1R1B; |
| cg07212818 | 37 | 11 | 638076 | DRD4 |
| cg08166587 | 37 | 12 | 2696048 | CACNA1C;; |
| cg26864301 | 37 | 7 | 1.51E+08 | KCNH2;KCNH2 |

**Discussion**

We have previously shown that psychological variables (CASI), (Chidambaran et al., European J Pain 2017; 21(7):1252-1265) (clinical variables and *OPRM1* promoter DNA methylation (Chidambaran et al., Pharmgenomics Pers Med 2017;10:157-168) associated with CPSP. In this study, we performed an EW AS of CPSP (acute or chronic perioperative pain) and CASI (anxiety sensitivity) in children undergoing spinal surgery and followed up on our findings with an integrative computational analysis to identify common, targetable pathways and transcription factors associated with significantly methylated CpG sites associated with CPSP and anxiety. Our findings open new avenues for personalized interventions based on epigenetics, and add to the emerging evidence linking epigenetic mechanisms to the development of chronic pain and psychological states. (Shimada-Sugimoto et al., Clin. Epigenetics 2017; 9(1): 6; Denk et al., Nature Neuroscience 2014; 17(2):192-200)

Epigenetic research into acute to chronic pain transitions (Bucheit T et al., Pain Medicine 2012; 13(11):1474-1490) is still in its infancy. To our knowledge, there are only a handful of clinical epigenetic studies in postsurgical patients. DNA methylation of the Secreted Protein, Acidic, Rich in Cysteine (*SPARC*) promoter was shown to play a role in chronic low-back pain related to degenerated intervertebral discs (Tajerian M et al.,; Molecular Pain 2011; 7:65-73). CpG methylation within the Tumor Necrosis Factor (*TNF*) gene promoter has also been identified as an additional mechanism through which *TNF* alters the risk for mild persistent breast pain after breast cancer surgery (Stephens KE et al., Cytokine 2017; 99:203-213). We previously reported on two CpG sites in an active regulatory region of the *OPRM1* gene that binds multiple transcription factors to be predictive of CPSP in another subset of the spine surgery cohort. (Chidambaran V et al., Pharmgenomics Pers. Med. 2017; 10:157-168). The present EWAS study provides further evidence for the role of epigenetics in CPSP.

Epigenome-based pathway analyses have been previously described using whole blood DNA in a large cohort of adults, with chronic widespread musculoskeletal pain (Livshits G et al., Pain 2017;158(6):1053-1062). They found that 6% of variance for the pain phenotype was explained by epigenetic factors, and showed enrichment for neurological pathways, including synaptic long-term depression, axonal guidance signaling, CREB, neuropathic pain signaling and melatonin signaling (Livshits G et al., Pain 2017;158(6):1053-1062). While some of the pathways are similar to what we have identified for CPSP, the differences may be reflective of differences in the nature of the pain and cohorts evaluated.

Of great interest is that the top canonical pathways common to both CPSP and CASI were identified to be the GABA receptor signaling and Dopamine-DARPP32 pathways. This is aligned with previous literature citing hypofunction of GABAergic inhibitory tone in the dorsal horn of the spinal cord as a key factor in central neuropathic pain after spinal cord injury (Drew GM et al., Pain 2004;109(3):379-388). Mechanisms proposed for GABAergic hypofunction include decreased number of GABA receptors (through apoptosis), downregulation of GABA synthesizing enzyme (GAD) and decreased GABA concentrations. Multiple *in vitro* and *in vivo* studies suggest the role of DNA methyltransferases in the epigenetic regulation of GABAergic gene expression in the cortex, striatum and hippocampus (Kadriu B et al., J. Comp. Neurol. 2012; 520(9):1951-1964). DNA epigenetic modifications of GABAergic interneurons in the basolateral amygdala have also been shown to be involved in the etiology of anxiety-like phenotype in prenatal stress mice, which could be reversed by demethylating agent, 5-Aza deoxycytidine (Zhu C et al., Int J Neuropharmacol 2018; 21(6):570-581). Our functional genomics analyses show that many of the CpG sites identified are located in regions of the brain marked by lysine 27 tri-methylation (H3K27me3), which is known to negatively regulate gene expression. Our study thus provides new evidence for DNA methylation as a mechanism for possibly reduced function of the GABA receptor pathway genes and its role in CPSP and anxiety pathogenesis.

Our findings are also aligned with postulated roles for the DARPP-32 dopamine pathway in the actions of drugs of abuse inflammatory states and psychiatric conditions like schizophrenia and bipolar disorder. DARPP-32 is a substrate of cAMP-dependent protein kinase (PKA) highly concentrated in dopamine-innervated brain areas, which functions as a PKA-regulated inhibitors of protein phosphatase-1 (PP1). The identification of epigenetic enrichment of this pathway is exciting because animal studies suggest a role for this phosphoprotein as an intracellular detector of convergent dopamine-1 receptor and N-methyl-D-aspartate (NMDA) receptor activation (Buesa I et al., Neuropharmacology 2006; 50(5): 585-594). These are target receptors for pain medications (opioids) and antipsychotic medications (for example haloperidol). Thus our studies indicate therapeutic interventions for CPSP based on epigenetic profile. A cyclin-dependent kinase 5 (Cdk5) inhibitor, roscovitine, was shown to decrease DARPP-32 phosphorylation (Wang CH et al., Acta Pharmacol. Sin. 2005; 26(1):46-50) and its intrathecal use decreased the formalin-induced nociceptive response in rats (Wang CH et al., Acta Pharmacol. Sin. 2005; 26(1):46-50) and remifentanil-induced hyperalgesia (Liu X et al., Brain Res Bull 2014;106:9-16). Dopamine is involved in reward mechanisms (Scott DJ et al., Arch. Gen. Psychiatry 2008; 65(2):220-231) and motivation to engage in pain self-management behaviors is an important predictor of adaptation/coping with acute pain. Accordingly, anxiety induced avoidance or lack of motivation (Navratilova E et al., PNAS USA 2012;109(50):20709-20713) is a plausible mechanism by which dopamine signaling might be a player in development of CPSP in the presence of anxiety.

Among the other top shared pathways, nitric oxide signaling (NOS) also deserves mention. This appears to be essential for neural plasticity and modulation of opioid action (Toda MDPDN et al., Anesthesiology 2009;110(1): 166-181). Nitric oxide formed by N-methyl-d-aspartate (NMDA)-receptor activation, may act at several levels of the nervous system to develop hyperexcitability, resulting in hyperalgesia or allodynia (Levy D et al., Pain Pract. 2004; 4(1):11-18). While it has been shown to be an analgesic (Pataki I et al., Eur J Pharmacol 1998; 357:2-3)157-162) and algesic (Hervera A et al., Molecular Pain 2011; 7:25-35) mediator at spinal, supraspinal and systemic sites in experimental animals, Pu et. al. postulated a dual-control mechanism composed of the excitatory NMDA and the inhibitory μ-opioid receptors in modulating cyclic GMP/nitric oxide release (Pu S et al. Endocrinology 1997; 138(4):1537-1543). Moreover, NOS also plays a role in morphine dependence and tolerance, which has been shown to be prevented using NOS inhibitors (Rahmati B et al., J Ethnopharmacol. 1997;199:39-51).

There is evidence from prior studies for the role of some of the other pathways we have identified to play a role in anxiety. Bioinformatics analysis of differently expressed microRNAs in anxiety disorder used for predicting target genes and functions using gene ontology and KEGG pathway analysis showed significant enrichment in several pathways related to neuronal brain functions such as the GnRH signaling pathway (Fan H et al., Zhonghua Yi Xue Yi Chuan Za Zhi 2015; 32(5):641-646). The Protein Kinase A (PKA) signaling pathway, closely related to the DARPP-32 pathway described above, is involved in neuronal plasticity in the amygdala, is responsible for amplification of anxiety behaviors in response to stressful stimuli. Results of clinical studies support the finding that alterations in PKA are associated with various anxiety, depression, and other psychiatric disorders (Keil MF et al., Front Endocrinol. (Lausanne) 2016; 7(83):1-8.

We have found interesting evidence based on Enrichr and Pinet enrichment analyses, including overlaps with LINCS knockdowns signatures for the overlaps with TF targets. They reveal several TFs with neuronal phenotypes as regulators of significant subsets of overlapping (common to both outcomes) genes. These include, but are not limited to, REST, TRIM28, POUSF1, NFE2L2, GATA2 and NANOG (Table 7, Fig. 4).

Table 7. Transcription factors and associated gene targets identified by Enrichr and Pinet analysis. Statistical significance of the association is indicated as p-value, or 'NS' for not significant. P-values were calculated using the hypergeometric test by overlapping our target genes over available datasets and identifying over-or-under representation at a selected statistical significance level.

| Transcription Factor | Associated Gene Targets | P value |
|---|---|---|
| TP53 (CHEA) | MAD1L1, CACNA1C, BNC2,CSMD1 | 0.0034 |
| REST (CHEA) | MAD1L1, DHX35, RIMS2, CDH13, GALNT9, CACNA1A, SPTBN4 | 0.0110 |
| POU5F1 (CHEA) | KCTD9, CBX1, MAD1L1 | 0.0142 |
| TRIM28 (ENCODE) | CBX1, PRIM2 | 0.0229 |
| TCF3 (CHEA) | CBX1, MAD1L1, EXT1, RADIL, CDH13 | 0.0445 |
| NFE2L2 (CHEA) | EXT1, FAM179A, DHX35, SDCCAG8, LMF1 | 0.0471 |
| GATA2 (CHEA) | CACNA1C, OBSCN, GALNT2, LMF1 | 0.0624 |
| AR (CHEA) | SCARA5, RIMS2, CDH13, CACNA1C, BNC2 | 0.0601 |
| PPARD (CHEA) | EXT1, SPTBN4 | 0.1064 |
| NANOG (CHEA) | KCTD9, CBX1, EXT1 | 0.1091 |
| KLF4 (CHEA) | KCTD9, CBX1, MAD1L1, TRIM27 | NS |
| REST (ENCODE) | OGDHL, RIMS2 | NS |

(continued)

| Transcription Factor | Associated Gene Targets | P value |
|---|---|---|
| FOXM1 (ENCODE) | KCTD9 | NS |
| SOX2 (CHEA) | EXT1, PRIM2, CDH13 | NS |
| RAD21 (ENCODE) | PRIM2, RADIL, NXN, SCARA5 | NS |
| RUNX1 (CHEA) | SDCCAG8, LMF1, LCP2, AK8 | NS |
| USF2 (ENCODE) | DHX35, SNX8, OGDHL | NS |
| FOXP2 (ENCODE) | EXT1 | NS |
| MYC (CHIA) | KCTD9, TRIM27 | NS |
| NRF1 (ENCODE) | KCTD9, CBX1, PRIM2, ERICH1, SDCCAG8 | NS |

NANOG is also associated with POU5F1, KLF4, etc. and its LINCS knockdowns are strongly positively correlated (in multiple cell lines) with SMAD1/2/3, POLR2A (and other units of PolIIa), EP300 and other putative TFs targeting the overlap genes, which adds supporting evidence for them working together. Analysis of overlaps with LINCS knockdowns reveals GABA receptor subunits and GPRC5C, among other potential positive upstream regulators of overlap genes (Table 8, Fig. 5). The latter (GPRC5C) is associated with activation of NANOG (Gonzales KA et al., Cell 2015; 162(3):564-579), which seems to be consistent with its and related TFs targets being among (in this case predicted to be positively regulated) the overlap genes.

Table 8. Signatures of Gene Knockdowns for Protein Kinases and Potential Downstream Targets Among Genes with DNA Methylation Associated with CPSP and the CASI

| Pathway | P Value | Gene List |
|---|---|---|
| ABL1_knockdown_96h_HEPG2 | .0003 | TNXB, NXN, OGDHL, TRIM27, ADAMTS8 |
| DAPK3_knockdown_96h_HA1E | .0003 | SPTBN4, GALNT2, NXN, OGDHL, ADAMTS8 |
| RARB_knockdown_96h_HA1E | .003 | EXT1 PRIM2, ZNF814, LMF1 |
| GPR31_knockdown_96h_PC3 | .003 | RIMS2, ZNF814, CACNA1C, ADAMTS8 |
| GABRG1_knockdown_96h_HCC515 | .003 | BNC2, ERICH1, ZNF814, CARD14 |
| RIPK2_knockdown_96h_HCC515 | .003 | EXT1, OBSCN, TNXB, TRIM27 |
| RIOK3_knockdown_96h_HA1E | .003 | SPTBN4, OBSCN, TNXB, ADAMTS8 |
| GABBR1_knockdown_96h_HA1E | .003 | OBSCN, NXN, ZNF814, TRIM27 |
| GPRC5C_knockdown_96h_PC3 | .003 | RIMS2, PRIM2, SPTBN4, CACNA1C |
| GPR151_knockdown_96h_A375 | .003 | EXT1, SPTBN4, ERICH1, CARD14 |
| ADRA2A_knockdown_96h_A375 | .003 | SPTBN4, ERICH1, GALNT2, CARD14 |
| CHRM3_knockdown_96h_PC3 | .003 | SPTBN4, OBSCN, ERICH1, CACNA1C |
| FLT3_knockdown_96h_HA1E | .003 | PRIM2, SPTBN4, NXN, ADAMTS8 |
| CSNK1G2_knockdown_96h_HA1E | .003 | PRIM2, NXN, OGDHL, ADAMTS8 |
| TNIK_knockdown_96h_HEPG2 | .003 | SPTBN4, GALNT2, NXN, OGDHL |

Note: LINCS L1000 kinase perturbations show genes identified in this study as undergoing epigenetic regulations (right column) being significantly downregulated by kinase knock-down signatures in the left column, thus representing potential downstream targets of the respective kinase signaling cascade. Note that, for each kinase, the corresponding cell line is also indicated, for example, GABRG1 knock-down in HCC515 cells in the fifth row.

Although this study utilized blood samples for measurement of DNA methylation status, instead of a more relevant target tissue such as brain tissue, the translational relevance of findings in easily available tissues such as blood cannot be overemphasized. Also, the ChIP assay findings show similar transcription factors at the identified CpG sites across tissues and regulatory regions in brain tissue areas relevant to pain, which may be indicative of methylation at these sites having an effect on expression. Moreover, methylation profiles derived from 12 tissues were compared in a previous study and found to be highly correlated between somatic tissues (Fan S et al., Biochem Biophy Res Commun 2009; 383(4):421-425). Davies et al. also reported that inter-individual variation in DNA methylation was reflected across brain and blood, indicating that peripheral tissues may have utility in studies of complex neurobiological phenotypes (Davies MN et al., Genome Biol. 2012; 13(6):R43). Our study is comprehensive in evaluating several relevant covariates with known influence on CPSP and anxiety sensitivity. Further work may involve evaluation in larger prospective cohorts and longitudinal evaluation of methylation changes after surgery.

DNA methylation is influenced by multiple modifiable factors such as diet, exercise, stress, and meditation. Therefore, understanding the shared role of epigenetic regulation of CPSP and anxiety opens new avenues of pain research. Our findings provide a basis for biopsychosocial profiles involved in CPSP and suggest consideration of behavioral and other pathway- targeted strategies, based on the individual's methylation profile at one or more of the CpG sites described here as determined from a blood sample (Niederberger E et al., Nature Reviews Neurology 2017;13(7):434-447). There is also promise from animal models for epigenetic modification to prevent the progression to chronic postsurgical pain (Denk F et al., Neuron 2012;73(3):435-444); Denk F et al., Nature Neuroscience 2014; 17(2):192-200) and use of demethylating drugs in other diseases (Saunthararajah Y et al., Brit J. Haematol. 2004; 126(5):629-636; Viet CT, Clinical Cancer Res. 2014; 20(18):4882-4893) for such therapies to be useful for the treatment of chronic pain. Recent advent of targeted epigenetic modification (Chen H et al., Nucleic Acids Res. 2014; 42(3):1563-1574) also provides hope for decreasing non-specific effects and poor delivery of epigenetic modulation to target cells and tissues, a major impediment to the development and clinical application of such analgesics.

Example 2: **Use of cytokines as biomarkers for susceptibility to pain**

The following additional experiments provide a basis for the use of cytokine expression in blood obtained from patients as a proxy for surgery related inflammation leading to CPSP. The data supports our hypothesis that pain is characterized by a heightened inflammatory responsiveness in susceptible patients and suggests that the immune system is "primed" in pain patients. Patients presenting with such a 'primed' immune system can be identified, for example, by assaying for perioperative pro-inflammatory cytokine levels, e.g., TNFα and IL1RA, and/or for lower levels of anti-inflammatory cytokines such as IL-4 in order to identify patients at risk of developing CPSP. At risk patients could be treated with one or more interventions aimed at decreasing risk of CPSP, for example treatment with an anti-inflammatory agent.

**Perioperative pro-inflammatory cytokine levels are higher in those who develop CPSP.** We also found that perioperative pro-inflammatory cytokine levels are higher in those who develop CPSP. Cytokine levels were measured in serum samples obtained at baseline and 4 time points within 2 hours of surgery (140 samples from 27 patients) using Human Cytokine/Chemokine Panel I (Milliplex® Immunology Panel). Average levels in patients who developed CPSP (N=14) and those who did not are plotted in Fig 6A. We found significantly increased pro-inflammatory cytokines such as tumor necrosis factor alpha (TNFα) and interleukin-1RA (IL1RA), as well as decreased levels of anti-inflammatory cytokines such as IL-4 in patients with CPSP, compared to controls. Additional differentially expressed cytokines were fractalkine, epidermal growth factor (EGF), FMS-like tyrosine kinase 3 ligand (FLT-3L), macrophage derived chemokine (MDC), interleukin-13 (IL-13), interleukin-8 (IL-8), and interleukin-4 (IL-4).

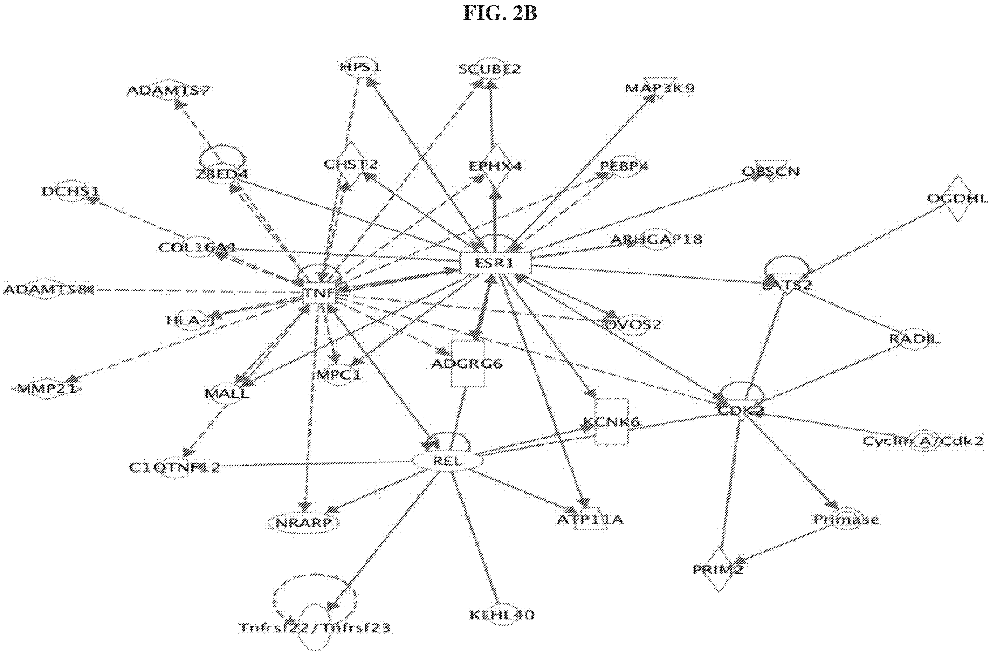

**Immune pathways are among the top canonical pathways enriched with DNAm associated with CPSP.** Our pilot EWAS in spine patients showed DNAm enrichment of PKCθ Signaling in T Lymphocytes (p<0.001) (overlap of HLA-A, PLCG2, CACNA1H, CACNA1C, CACNA1A, HLA-DRB5, LCP2, CAMK2B) and FcγRIIB Signaling in B Lymphocytes (PLCG2, CACNA1H, CACNA1C, CACNA1A) (p=0.015). Gene-gene interaction network enrichment analysis revealed participation of pathways in cell signaling, molecular transport, immune responses, metabolism and neurological diseases (p-value < 10-8) (Fig. 2A) in CPSP, with several target molecules. This is shown graphically in Fig. 2B.

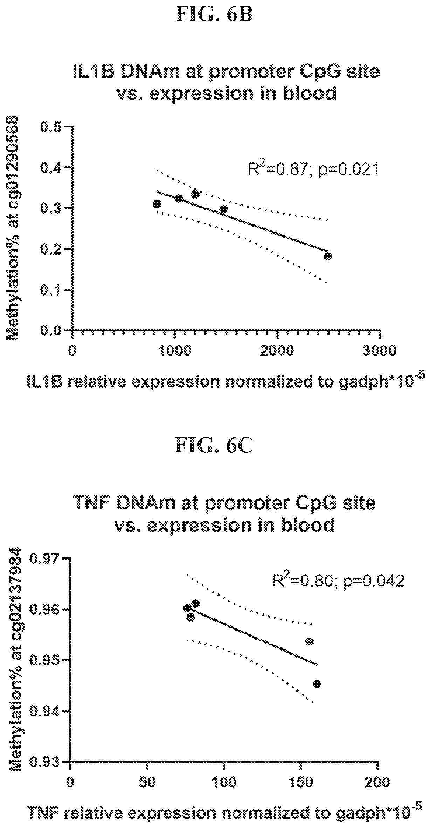

**DNAm of inflammatory genes correlates with decreased gene expression in blood**. RT-PCR and pyrosequencing were used to examine DNAm and gene expression in blood for cytokine genes whose cytokines were elevated in CPSP. We found negative correlation trends between promoter DNAm and gene expression for cytokine genes. For example, the negative correlation between IL1B expression and DNA methylation (DNAm) is shown in Fig 6B and that of TNFalpha expression versus DNA methylation is shown in Fig. 6C. Additionally, CPSP was nominally associated with decreased CpG DNAm, consistent with increased cytokines.

## Claims

1. A therapeutic agent which is a demethylating agent for use in a method for the prophylaxis or treatment of perioperative pain in a human patient identified as susceptible to perioperative pain,
   wherein the patient has been identified as susceptible to perioperative pain by a method comprising assaying, *in vitro,* a biological sample from the patient to determine the DNA methylation status of a plurality of CpG sites in a plurality of genes of a molecular signaling pathway selected from a GABA receptor signaling pathway and a dopamine-DARPP32 feedback in cAMP signaling pathway, or both, wherein the plurality of genes includes at least two genes selected from the group consisting of either ABAT, ADCY5, CACNA1H, CACNA1C, GABBR1, KCNH2, CACNA1A or PPP1R1B, ADCY5, PLCG2, CAMKK1, CACNA1C, DRD4, CACNA1A and wherein having a DNA methylation status of 'methylated' is identified as a patient susceptible to perioperative pain; and wherein the perioperative pain is chronic postsurgical pain.

2. The therapeutic agent for the use of claim 1, wherein the demethylating agent is selected from procaine, zebularine and decitabine, or a combination of two or more of the foregoing.

3. A method for identifying a human patient who is susceptible to perioperative pain, the method comprising assaying, *in vitro,* a biological sample from the patient to determine the DNA methylation status of a plurality of CpG sites in a plurality of genes of a molecular signaling pathway selected from a GABA receptor signaling pathway and a dopamine-DARPP32 feedback in cAMP signaling pathway, or both, wherein the plurality of genes includes at least two genes selected from the group consisting of either ABAT, ADCY5, CACNA1H, CACNA1C, GABBR1, KCNH2, CACNA1A or PPP1R1B, ADCY5, PLCG2, CAMKK1, CACNA1C, DRD4, CACNA1A and wherein having a DNA methylation status of 'methylated' is identified as a patient susceptible to perioperative pain; and wherein the perioperative pain is chronic postsurgical pain.

4. The therapeutic agent for the use of claim 1 or 2, or the method of claim 3, wherein the biological sample is a whole blood sample.

5. The therapeutic agent for the use of any one of claims 1-2 or 4, or the method of any one of claims 2-4, wherein the biological sample is assayed by a method comprising isolation of genomic DNA from the biological sample, optionally wherein the biological sample is assayed by a method comprising, or further comprising, pyrosequencing, and further optionally wherein the pyrosequencing comprises two or more rounds of a polymerase chain reaction.

6. The therapeutic agent for the use of any one of claims 1-2 or 4-5, or the method of any one of claims 2-5, wherein the patient is a female patient, and/or wherein the patient is white.

**Patentansprüche**

1. Therapeutikum, das ein Demethylierungsmittel ist, zur Verwendung in einem Verfahren für die Prophylaxe oder Behandlung von perioperativem Schmerz bei einem menschlichen Patienten, der als anfällig für perioperativen Schmerz identifiziert wurde,
wobei der Patient als anfällig für perioperativen Schmerz durch ein Verfahren identifiziert wurde, umfassend ein Untersuchen, *in vitro,* einer biologischen Probe von dem Patienten, um den DNA-Methylierungsstatus einer Vielzahl von CpG-Stellen in einer Vielzahl von Genen eines molekularen Signalwegs, ausgewählt aus einem GABA-Rezeptor-Signalweg und einem Dopamin-DARPP32-Feedback in dem cAMP-Signalweg, oder beiden, zu bestimmen, wobei die Vielzahl von Genen mindestens zwei Gene einschließt, ausgewählt aus der Gruppe, bestehend aus entweder ABAT, ADCY5, CACNA1H, CACNA1C, GABBR1, KCNH2, CACNA1A oder PPP1R1B, ADCY5, PLCG2, CAMKK1, CACNA1C, DRD4, CACNA1A, und wobei ein Aufweisen eines DNA-Methylierungsstatus als "methyliert" als ein Patient identifiziert wird, der anfällig für perioperativen Schmerz ist; und wobei der perioperative Schmerz ein chronischer postoperativer Schmerz ist.

2. Therapeutikum für die Verwendung nach Anspruch 1, wobei das Demethylierungsmittel aus Procain, Zebularin und Decitabin oder einer Kombination aus zwei oder mehr der Vorgenannten ausgewählt ist.

3. Verfahren zum Identifizieren eines menschlichen Patienten, der anfällig für perioperativen Schmerz ist, wobei das Verfahren das Untersuchen, *in vitro,* einer biologischen Probe von dem Patienten umfasst, um den DNA-Methylierungsstatus einer Vielzahl von CpG-Stellen in einer Vielzahl von Genen eines molekularen Signalwegs, ausgewählt aus einem GABA-Rezeptor-Signalweg und einem Dopamin-DARPP32-Feedback in dem cAMP-Signalweg, oder beiden, zu bestimmen, wobei die Vielzahl von Genen mindestens zwei Gene einschließt, ausgewählt aus der Gruppe, bestehend aus entweder ABAT, ADCY5, CACNA1H, CACNA1C, GABBR1, KCNH2, CACNA1A oder PPP1R1B, ADCY5, PLCG2, CAMKK1, CACNA1C, DRD4, CACNA1A, und wobei ein Aufweisen eines DNA-Methylierungsstatus als "methyliert" als ein Patient identifiziert wird, der anfällig für perioperativen Schmerz ist; und wobei der perioperative Schmerz ein chronischer postoperativer Schmerz ist.

4. Therapeutikum für die Verwendung nach Anspruch 1 oder 2 oder Verfahren nach Anspruch 3, wobei die biologische Probe eine Vollblutprobe ist.

**5.** Therapeutikum für die Verwendung nach einem der Ansprüche 1 bis 2 oder 4 oder Verfahren nach einem der Ansprüche 2 bis 4, wobei die biologische Probe durch ein Verfahren untersucht wird, das eine Isolierung genomischer DNA aus der biologischen Probe umfasst, optional, wobei die biologische Probe durch ein Verfahren untersucht wird, das Pyrosequenzierung umfasst oder ferner umfasst, und weiter optional, wobei die Pyrosequenzierung zwei oder mehr Runden einer Polymerasekettenreaktion umfasst.

**6.** Therapeutikum für die Verwendung nach einem der Ansprüche 1 bis 2 oder 4 bis 5 oder Verfahren nach einem der Ansprüche 2 bis 5, wobei der Patient eine weibliche Patientin ist und/oder wobei der Patient weiß ist.

**Revendications**

**1.** Agent thérapeutique qui est un agent déméthylant destiné à être utilisé dans un procédé pour la prophylaxie ou le traitement de douleur péri-opératoire chez un patient humain identifié comme étant sensible à la douleur péri-opératoire,
où le patient a été identifié comme étant sensible à la douleur péri-opératoire par un procédé comprenant l'analyse, *in vitro,* d'un échantillon biologique du patient afin de déterminer l'état de méthylation de l'ADN d'une pluralité de sites CpG dans une pluralité de gènes d'une voie de signalisation moléculaire choisie parmi une voie de signalisation du récepteur GABA et une voie de signalisation de la rétroaction de la dopamine-DARPP32 dans l'AMPc, ou les deux, où la pluralité de gènes comporte au moins deux gènes choisis dans le groupe constitué soit d'ABAT, ADCY5, CACNA1H, CACNA1C, GABBR1, KCNH2, CACNA1A, soit de PPP1R1B, ADCY5, PLCG2, CAMKK1, CACNA1C, DRD4, CACNA1A et où le fait d'avoir un état de méthylation de l'ADN 'méthylé' est identifié comme un patient sensible à la douleur péri-opératoire ; et où la douleur péri-opératoire est une douleur post-chirurgicale chronique.

**2.** Agent thérapeutique pour l'utilisation selon la revendication 1, où l'agent déméthylant est choisi parmi procaïne, zébularine et décitabine, ou une combinaison de deux ou plus de ce qui précède.

**3.** Procédé d'identification d'un patient humain qui est sensible à la douleur péri-opératoire, le procédé comprenant l'analyse, *in vitro,* d'un échantillon biologique du patient afin de déterminer l'état de méthylation de l'ADN d'une pluralité de sites CpG dans une pluralité de gènes d'une voie de signalisation moléculaire choisie parmi une voie de signalisation du récepteur GABA et une voie de signalisation de la rétroaction de la dopamine-DARPP32 dans l'AMPc, ou les deux, où la pluralité de gènes comporte au moins deux gènes choisis dans le groupe constitué soit d'ABAT, ADCY5, CACNA1H, CACNA1C, GABBR1, KCNH2, CACNA1A, soit de PPP1R1B, ADCY5, PLCG2, CAMKK1, CACNA1C, DRD4, CACNA1A et où le fait d'avoir un état de méthylation de l'ADN 'méthylé' est identifié comme un patient susceptible sensible à la douleur péri-opératoire ; et où la douleur péri-opératoire est une douleur post-chirurgicale chronique.

**4.** Agent thérapeutique pour l'utilisation selon la revendication 1 ou 2, ou procédé selon la revendication 3, où l'échantillon biologique est un échantillon de sang total.

**5.** Agent thérapeutique pour l'utilisation selon l'une quelconque des revendications 1 et 2 ou 4, ou procédé selon l'une quelconque des revendications 2 à 4, où l'échantillon biologique est analysé par un procédé comprenant l'isolement de l'ADN génomique de l'échantillon biologique, où l'échantillon biologique est facultativement analysé par un procédé comprenant, ou comprenant en outre, le pyroséquençage, et en outre où le pyroséquençage comprend facultativement deux cycles ou plus d'une réaction en chaîne par polymérase.

**6.** Agent thérapeutique pour l'utilisation selon l'une quelconque des revendications 1 et 2 ou 4 et 5, ou procédé selon l'une quelconque des revendications 2 à 5, où le patient est une femme, et/ou où le patient est blanc.

## FIG. 1

```
┌─────────────────────────────────────────────────────────────────┐
│ Quality Control: Swan normalized beta, combat correct for batch   │
│                        56 samples                                 │
└─────────────────────────────────────────────────────────────────┘
                              │
                              ▼          One sample did not pass QC
        ┌──────────────────────────────────────────┐
        │ 842148 CpG sites (55 samples)            │
        └──────────────────────────────────────────┘
                              │
                              ▼
    ┌──────────────────────────────────────────────────────┐
    │ Model DNAm = CPSP/CASI + age + sex + race + SVs.      │
    │        Beta and M values were modeled                 │
    └──────────────────────────────────────────────────────┘
                              │
                              ▼
┌──────────────────────────────────────────────────────────────────┐
│ CpG sites with statistical significance (p value ≤ 0.05 in both   │
│           beta and M models)                                      │
│        and difference ≥ 0.05 were selected                        │
│  (2128 sites for CPSP and 21,982 sites for CASI)                  │
└──────────────────────────────────────────────────────────────────┘
                              │
                              ▼
        ┌──────────────────────────────────────────┐
        │      Association regression models:       │
        │      Model CPSP = DNAm + age + CASI        │
        │    Model CASI=PCS-P + DNAm + Diazepam      │
        └──────────────────────────────────────────┘
                              │
                              ▼
┌──────────────────────────────────────────────────────────────────┐
│ Sites significantly associated with CPSP and CASI (p<=0.05)       │
│        637 sites for CPSP; 2,445 sites for CASI                   │
└──────────────────────────────────────────────────────────────────┘
              │                                    │
              ▼                                    ▼
    ┌─────────────────────────┐        ┌─────────────────────────┐
    │ Ingenuity Pathway       │        │ Functional epigenomics  │
    │ Analysis                │        │                         │
    └─────────────────────────┘        └─────────────────────────┘
```

FIG. 2A

FIG. 2B

FIG. 3B

EP 3 813 814 B1

FIG. 4

## FIG. 5

FIG. 6A

Significantly (p<0.05) different marker
levels between the 2 groups

## FIG. 6B

### IL1B DNAm at promoter CpG site vs. expression in blood

$R^2=0.87; p=0.021$

Methylation% at cg01290568

IL1B relative expression normalized to gadph*$10^{-5}$

## FIG. 6C

### TNF DNAm at promoter CpG site vs. expression in blood

$R^2=0.80; p=0.042$

Methylation% at cg02137984

TNF relative expression normalized to gadph*$10^{-5}$

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 631622, Levenson

### Non-patent literature cited in the description

- **MACRAE WA**. *Brit. J. Anaesthesia*, 2008, vol. 101 (1), 77-8
- **RABBITTS JA et al.** *J. of Pain*, 2017, vol. 18 (6), 605-614
- **CHIDAMBARAN V et al.** *Eur. J. of Pain*, 2017, vol. 21 (7), 1252-1265
- **LANDMAN Z et al.** *Spine*, 2011, vol. 36 (10), 825-829
- **SIEBERG CB et al.** *J. of Pain*, 2013, vol. 14 (12), 1694-1702
- **JAMES SK**. *Brit. J. of Pain*, vol. 11 (4), 178-185
- **YOUNG EE et al.** *J. Med. Genet.*, 2012, vol. 49 (1), 1-9
- **ANGST MS et al.** *Pain*, vol. 153 (7), 1397-1409
- **NORBURY TA et al.** *Brain*, 2007, vol. 130 (11), 3014-3049
- **KATZ J**. *Pain*, 2012, vol. 153 (3), 505-506
- **KIM H et al.** *J. Pain*, vol. 10 (7), 663-693
- **SADHASIVAM S et al.** *Pharmacogenomics*, 2012, vol. 13 (15), 1719-1740
- **BRANFORD R et al.** *Clin. Genet.*, 2012, vol. 82 (4), 301-310
- **WALTER C et al.** *Pain*, 2009, vol. 146 (3), 270-275
- **DENK F et al.** *Neuron*, 2012, vol. 73 (3), 435-444
- **HETTEMA JM et al.** *Arch. Gen. Psychiatry*, 2005, vol. 62 (2), 182-189
- **CROW M et al.** *Genome Med.*, 2013, vol. 5 (2), 12-21
- **DOERHING A et al.** *Eur. J. Pain*, 2011, vol. 15 (1), 11-16
- **BUCHEIT T et al.** *Pain Medicine*, 2012, vol. 13 (11), 1474-1490
- **CHORBOV VM et al.** *J. Opioid Manag.*, 2011, vol. 7 (4), 258-264
- **ZORINA-LICHENWALTER K et al.** *Neuroscience*, 2016, vol. 338, 36-62
- **SHIMADA-SUGIMOTO M et al.** *Clinical Epigenetics*, 2017, vol. 9 (1), 6-16
- **CHIDMBARAN, V. et al.** *Pharmacogenomics and Personalized Medicine*, 2017, vol. 10, 157-168
- **FAN H et al.** *Zhonghua Yi Xue Yi Chuan Xue Za Zhi*, 2015, vol. 32 (5), 641-645
- **DAVIES MN et al.** *Genome Biol.*, 2012, vol. 13 (6), R43
- **FAN S et al.** *Biochem. Biophys. Res. Comm.*, 2009, vol. 383 (4), 421-425
- **DELANEY et al.** In: Methods Mol. Biol.. Springer Humana Press, 2015, vol. 1343, 249-264
- **GRACA I et al.** *Curr. Pharmacol. Design.*, 2014, vol. 20, 1803-11
- **CHARBORD J et al.** *Stem Cells*, 2013, vol. 20, 1803-1811
- **SINGLETON et al.** Dictionary of Microbiology and Molecular Biology. J. Wiley & Sons, 2001
- **MARCH**. Advanced Organic Chemistry Reactions, Mechanisms and Structure. J. Wiley & Sons, March 2001
- **SAMBROOK ; RUSSEL**. Molecular Cloning: A Laboratory Manual. Cold Spring Harbor Laboratory Press, 2001
- **GERBERSHAGEN et al.** *Brit. J. Anaesth.*, 2011, vol. 107 (4), 619-629
- **RABIAN et al.** *J. Clin. Child Psychol.*, 1999, vol. 28 (1), 105-112
- **SILVERMAN et al.** *J. Abnorm. Psychol.*, 2003, vol. 112 (3), 364-374
- **CHIDAMBARAN et al.** *Pharmgenomics Pers Med.*, 2017, vol. 10, 157-168
- **PAGE et al.** *J. Pain Res*, 2013, vol. 6, 167-180
- **DU P et al.** *BMC Bioinformatics*, 2010, vol. 11, 587
- **CHIDAMBARAN V et al.** *Eur. J. Pain*, 2017, vol. 21 (7), 1252-1265
- **CONSORTIUM EP et al.** *Nature*, 2012, vol. 489 (7414), 57-74
- **BERNSTEIN BE et al.** *Nat. Biotech.*, 2010, vol. 28 (10), 1045-1048
- **LIU T et al.** *Genome Biol.*, 2011, vol. 12 (8), R83
- **GRIFFON A et al.** *Nucl. Acids Res.*, 2015, vol. 43 (4), e27

- **ERNST J et al.** *Nature*, 2011, vol. 473 (7345), 43-49
- **HARLEY JB et al.** *Nature Neurosci.*, 2018, vol. 17 (2), 192-200
- **KOLETI A et al.** *Nucl. Acids Res.*, 2018, vol. 46 (D1), D558-D566
- **KEENAN AB et al.** *Cell System.*, 2018, vol. 6 (1), 13-24
- **CHIDAMBARAN et al.** *European J Pain*, 2017, vol. 21 (7), 1252-1265
- **CHIDAMBARAN et al.** *Pharmgenomics Pers Med*, 2017, vol. 10, 157-168
- **SHIMADA-SUGIMOTO et al.** *Clin. Epigenetics*, 2017, vol. 9 (1), 6
- **DENK et al.** *Nature Neuroscience*, 2014, vol. 17 (2), 192-200
- **TAJERIAN M et al.** *Molecular Pain*, 2011, vol. 7, 65-73
- **STEPHENS KE et al.** *Cytokine*, 2017, vol. 99, 203-213
- **CHIDAMBARAN V et al.** *Pharmgenomics Pers. Med.*, 2017, vol. 10, 157-168
- **LIVSHITS G et al.** *Pain*, 2017, vol. 158 (6), 1053-1062
- **DREW GM et al.** *Pain*, 2004, vol. 109 (3), 379-388
- **KADRIU B et al.** *J. Comp. Neurol.*, 2012, vol. 520 (9), 1951-1964
- **ZHU C et al.** *Int J Neuropharmacol*, 2018, vol. 21 (6), 570-581
- **BUESA I et al.** *Neuropharmacology*, 2006, vol. 50 (5), 585-894
- **WANG CH et al.** *Acta Pharmacol. Sin.*, 2005, vol. 26 (1), 46-50
- **LIU X et al.** *Brain Res Bull*, 2014, vol. 106, 9-16
- **SCOTT DJ et al.** *Arch. Gen. Psychiatry*, 2008, vol. 65 (2), 220-231
- **NAVRATILOVA E et al.** *PNAS USA*, 2012, vol. 109 (50), 20709-20713
- **TODA MDPDN et al.** *Anesthesiology*, 2009, vol. 110 (1), 166-181
- **LEVY D et al.** *Pain Pract.*, 2004, vol. 4 (1), 11-18
- **PATAKI I et al.** *Eur J Pharmacol*, 1998, vol. 357 (2-3), 157-162
- **HERVERA A et al.** *Molecular Pain*, 2011, vol. 7, 25-35
- **PU S et al.** *Endocrinology*, 1997, vol. 138 (4), 1537-1543
- **RAHMATI B et al.** *J Ethnopharmacol.*, 1997, vol. 199, 39-51
- **FAN H et al.** *Zhonghua Yi Xue Yi Chuan Za Zhi*, 2015, vol. 32 (5), 641-646
- **KEIL MF et al.** *Front Endocrinol. (Lausanne)*, 2016, vol. 7 (83), 1-8
- **GONZALES KA et al.** *Cell*, 2015, vol. 162 (3), 564-579
- **FAN S et al.** *Biochem Biophy Res Commun*, 2009, vol. 383 (4), 421-425
- **NIEDERBERGER E et al.** *Nature Reviews Neurology*, 2017, vol. 13 (7), 434-447
- **DENK F et al.** *Nature Neuroscience*, 2014, vol. 17 (2), 192-200
- **SAUNTHARARAJAH Y et al.** *Brit J. Haematol.*, 2004, vol. 126 (5), 629-636
- **VIET CT.** *Clinical Cancer Res.*, 2014, vol. 20 (18), 4882-4893
- **CHEN H et al.** *Nucleic Acids Res.*, 2014, vol. 42 (3), 1563-1574